(19) Europäisches Patentamt / European Patent Office / Office européen des brevets

(11) **EP 2 912 100 B1**

(12) **EUROPÄISCHE PATENTSCHRIFT**

(45) Veröffentlichungstag und Bekanntmachung des Hinweises auf die Patenterteilung:
**05.09.2018 Patentblatt 2018/36**

(21) Anmeldenummer: **13780362.3**

(22) Anmeldetag: **24.10.2013**

(51) Int Cl.:
*C08H 8/00* (2010.01)       *B27K 5/00* (2006.01)
*B27N 1/00* (2006.01)       *B27N 3/02* (2006.01)
*B27N 3/04* (2006.01)       *C12N 1/04* (2006.01)
*C12N 1/36* (2006.01)       *C21C 5/00* (2006.01)
*C12R 1/00* (2006.01)

(86) Internationale Anmeldenummer:
**PCT/EP2013/072304**

(87) Internationale Veröffentlichungsnummer:
**WO 2014/064209 (01.05.2014 Gazette 2014/18)**

(54) **VERFAHREN ZUR HERSTELLUNG EINES HOLZWERKSTOFFS**

METHOD FOR THE PRODUCTION OF WOOD MATERIALS

PROCÉDÉ DE FABRICATION DE MATÉRIAUX EN BOIS

(84) Benannte Vertragsstaaten:
**AL AT BE BG CH CY CZ DK EE ES FI FR GB GR HR HU IE IS IT LI LT LU LV MC MK MT NL NO PL PT RO RS SE SI SK SM TR**

(30) Priorität: **24.10.2012 DE 102012020842**

(43) Veröffentlichungstag der Anmeldung:
**02.09.2015 Patentblatt 2015/36**

(73) Patentinhaber: **Fritz Egger GmbH & Co. OG 6380 St. Johann in Tirol (AT)**

(72) Erfinder:
• **KUNCINGER, Thomas A-1200 Wien (AT)**
• **GRADINGER, Cornelia A-2320 Schwechat (AT)**
• **STRATEV, Daniel A-1140 Wien (AT)**

(74) Vertreter: **Cohausz & Florack Patent- & Rechtsanwälte Partnerschaftsgesellschaft mbB Bleichstraße 14 40211 Düsseldorf (DE)**

(56) Entgegenhaltungen:
EP-A1- 2 138 528          WO-A1-2006/039914
DE-A1-102006 057 566

• **STRATEV, D.; GRADINGER, C.; TERS, T.; FACKLER, K.; KUNCINGER, T.; SREBOTNIK, E.: "Fungal pretreatment of pine wood to reduce the emission of volatile organic compounds", HOLZFORSCHUNG, 2011, XP009175954, in der Anmeldung erwähnt**
• **ANNE KALLIOINEN ET AL: "Effects of bacterial treatments on wood extractives", JOURNAL OF BIOTECHNOLOGY, Bd. 103, Nr. 1, 1. Juni 2003 (2003-06-01), Seiten 67-76, XP055099967, ISSN: 0168-1656, DOI: 10.1016/S0168-1656(03)00051-8**
• **T. A. BURNES ET AL: "Bacterial Biodegradation of Extractives and Patterns of Bordered Pit Membrane Attack in Pine Wood", APPLIED AND ENVIRONMENTAL MICROBIOLOGY, Bd. 66, Nr. 12, 1. Dezember 2000 (2000-12-01), Seiten 5201-5205, XP055100019, ISSN: 0099-2240, DOI: 10.1128/AEM.66.12.5201-5205.2000**
• **Anne Kallioinen ET AL: "Effects of bacterial treatments on wood extractives", Journal of Biotechnology, vol. 103, no. 1, 1 June 2003 (2003-06-01), pages 67-76, XP055099967, ISSN: 0168-1656, DOI: 10.1016/S0168-1656(03)00051-8**
• **J. Dorado ET AL: "Degradation of lipophilic wood extractive constituents in Pinus sylvestris by the white-rot fungi Bjerkandera sp. and Trametes versicolor", WOOD SCIENCE AND TECHNOLOGY., vol. 35, no. 1-2, 19 April 2001 (2001-04-19), pages 117-125, XP055367975, DE ISSN: 0043-7719, DOI: 10.1007/s002260000077**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Bemerkungen:
Die Akte enthält technische Angaben, die nach dem Eingang der Anmeldung eingereicht wurden und die nicht in dieser Patentschrift enthalten sind.

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft ein Verfahren zur Herstellung eines Holzwerkstoffs sowie ein Mikroorganismen enthaltendes Medium zur Verringerung des Gehalts an Holzextraktstoff, Vorläufersubstanzen von VOC und/oder VOC in einem Material auf Basis von Cellulose, insbesondere Holz und/oder einem Holzwerkstoff. Die Erfindung betrifft ferner einen Holzwerkstoff und die Verwendung eines Mikroorganismen enthaltenden Mediums zur Verringerung des Gehalts an Holzextraktstoff, Vorläufersubstanzen von VOC und/oder VOC in Holz oder einem Holzwerkstoff.

Verfahren in denen der Gehalt an Holzextraktstoff und/oder flüchtigen organischen Verbindungen (Englisch: volatile organic compounds, VOC) oder deren Vorläufersubstanzen in Cellulose enthaltenden Materialien reduziert wird, sind grundsätzlich bekannt.

Holzextraktstoff ist ein Gemisch aus verschiedenen Holzinhaltsstoffen, die in Lösungsmitteln, insbesondere in polaren und/oder apolaren organischen Lösungsmitteln und/oder Wasser löslich sind, beispielsweise in Wasser, Alkohol, Benzol, Hexan, Cyclohexan, Ether und/oder Aceton. Holzextraktstoffe, die gewöhnlich etwa 3 % bis 10 % des Holzgewichts ausmachen, enthalten beispielsweise niedermolekulare Kohlenwasserstoffe, Terpene, aromatische und aliphatische Säuren, Alkohole, Tannine, Farbstoffe, Proteine, Alkaloide und/oder lösliche Lignine. Insbesondere enthält Holzextraktstoff typischerweise auch VOC und/oder VOC-Vorläufersubstanzen.

Holzextraktstoffe können die Eigenschaften von Materialien, die in der holzverarbeitenden Industrie eingesetzt werden, wie beispielsweise die Oberflächeneigenschaften des Holzes sowie dessen Trocknungs- oder Leimungsverhalten, wesentlich verschlechtern. Folglich besteht ein Bedürfnis, die Menge an Holzextraktstoff in Cellulose enthaltenden Materialien, wie Holz (insbesondere Massivholz), Holzwerkstoffen und deren Vor- oder Zwischenprodukten (insbesondere Holzstrands, Holzspäne oder Holzfasern) beispielsweise für die Holzplattenherstellung zu reduzieren.

Das Vorkommen von Holzextraktstoffen und insbesondere den darin enthaltenen VOC ist aber auch aus anderen Gründen nachteilig. So werden in der Wärme, beispielsweise in Trocknungsschritten, wie sie üblicherweise in der Holz verarbeitenden Industrie durchgeführt werden, VOC Vorläufersubstanzen, wie beispielsweise die in den Holzextraktstoffen vorkommenden ungesättigten Fette oder Harzsäuren, durch thermische Induktion oxidiert, wobei VOC gebildet werden. Zusätzlich hierzu sind auch von Natur aus zahlreiche VOC, wie beispielsweise solche aus der Substanzklasse der Terpene, in Holzextraktstoffen enthalten. Die Freisetzung von VOC, und insbesondere von kurzkettigen Aldehyden, führt bei Holzwerkstoffen häufig zur Entstehung eines unangenehmen Geruchs im Endprodukt.

Eine der Hauptursachen der VOC-Entstehung in Cellulose enthaltenden Materialien ist der chemische, insbesondere der oxidative Abbau von Holzextraktstoffen. Weitere Quellen für VOC können der thermische und/oder chemische Abbau von Lignin, Hemicellulose bzw. Cellulose und/oder von Harzen sein. Die Freisetzung von VOC wird ferner auch durch eine Vergrößerung der Partikeloberfläche bei der Verarbeitung von Cellulose enthaltenden Produkten begünstigt.

Neben bindemittelseitig verursachten Formaldehydemissionen spielen VOC-Emissionen eine zunehmende Rolle bei der Bewertung der Raumbelastungen von Bauteilen und Werkstoffen aus Cellulose enthaltenden Materialien. Problematisch ist hierbei insbesondere die Freisetzung von Monoterpenen, Sesquiterpenen, Phenolen (insbesondere Guajakol) sowie niedermolekularen aliphatischen Alkoholen und/oder Aldehyden (insbesondere Heptanol und Hexanal), die von Cellulose enthaltenden Materialien emittiert werden. Die Emission von VOC unterliegt aufgrund befürchteter Gesundheitsgefährdungen zunehmend Restriktionen.

Aus diesem Grund existiert ein großes Bedürfnis in der holzverarbeitenden Industrie, die VOC-Emissionen von Cellulose enthaltenden Werkstoffen und Produkten zu reduzieren.

Es sind eine Reihe physiko-chemischer Verfahren zum Entfernen von Holzextraktstoffen oder zur Verringerung des VOC-Gehalts bekannt.

EP 2 181 818 A2 beschreibt u.a. ein Verfahren zur Herstellung von Holzfaserwerkstoffen mit verringerter VOC-Emission unter Einsatz einer Formulierung, die mindestens eine Verbindung zur Einstellung eines neutralen bis basischen pH-Wertes und mindestens einen Komplexbildner und als optionale weitere Komponente Antioxidantien zum Oxidationsschutz von Fetten enthält.

WO 2006/039914 A1 beschreibt den Einsatz von Alkalihydroxiden (speziell von NaOH), Alkalicarbonaten (speziell von $Na_2CO_3$), Alkalisulfiten (speziell von $Na_2SO_3$), Alkaliphosphaten (speziell von $Na_3PO_4$), Mischungen der vorgenannten Verbindungen sowie von Ammoniakgas ($NH_3$) zur Verminderung der Emissionen von VOC aus Holzwerkstoffen, vor allem aus OSB.

WO 2006/032267 A1 beschreibt u. a. den Einsatz alkalischer Verbindungen zur Herstellung emissionsgeminderter OSB aus fettreichen Nadelhölzern.

Aus der WO 2009/021702 A1 ist ein Behandlungsverfahren zur Reduktion der VOC und Formaldehyd Emission in Holzwerkstoffen bekannt, bei dem eine Mischung aus i) Hydrogensulfitsalz; ii) Sulfitsalz; sowie iii) gegebenenfalls Harnstoff und Harnstoffderivate und iv) gegebenenfalls Alkali-, Erdalkali- oder Ammoniumhydroxid eingesetzt wird.

Aus der EP 1 852 231 A2 ist ein Verfahren zur Herstellung von Holzwerkstoffen mit verminderter VOC Emission bekannt, bei welchem die VOCs mit einer Komponente A, die beispielsweise Phenol oder Resorcin sein kann, in Anwesenheit einer Komponente B, nämlich einer starken Säure oder eines Friedel-Crafts-Katalysators, zu höher molekularen Verbindungen umgesetzt werden, die nicht mehr flüchtig sind.

In jüngerer Zeit wurden ferner verschiedene biotechnologische Verfahren, insbesondere unter Verwendung von Enzymen, zum Entfernen von Holzextraktstoffen oder zur Verringerung des VOC-Gehalts vorgeschlagen. Enzymatische Verfahren weisen gegenüber den oben genannten physiko-chemischen Verfahren den Vorteil auf, dass sie im Allgemeinen wesentlich selektiver sind, weniger unerwünschte Nebenprodukte liefern und üblicherweise unter schonenderen Bedingungen durchgeführt werden können.

Nachteilig an enzymatischen Verfahren ist jedoch, dass die gereinigten Enzyme aufwändig in der Herstellung und daher teuer sind und meist nur unter bestimmten Reaktionsbedingungen optimale Ergebnisse zeigen. Ferner ist an dem Einsatz von gereinigten Enzymen nachteilig, dass diese ihren Einsatz in wässrigem Milieu bei Raumtemperatur oder höheren Temperaturen meist nicht überstehen und daher eine Wiederverwendung nicht sinnvoll ist. Schließlich ist bei den zur VOC-Reduktion eingesetzten Enzymen beobachtet worden, dass diese wegen ihrer hohen Substratspezifität nur zum Abbau von bestimmten VOC-Spezies führen, während der Gesamt VOC-Gehalt nicht ausreichend reduziert wird.

In den aus dem Stand der Technik bekannten biotechnologischen Verfahren zum Entfernen von Holzextraktstoffen werden üblicherweise bestimmte Mikroorganismen oder bestimmte, von Mikroorganismen hergestellte Enzyme verwendet.

So wurden beispielsweise albinotische Mutanten von *sap stain fungi* (hauptsächlich *Ophiostoma spp.*), die inzwischen im Handel erhältlich sind, und einige Weißfäulepilze (white rot fungi) im Hinblick auf ihre Fähigkeit, lipophile Verbindungen während der Lagerung abzubauen, getestet (M.J. Martinez-Inigo, et al., Time course of fungal removal of lipophilic extractives from eucalyptus globulus wood, Journal of Biotechnology 2000, 84, 119-126; M.J. Martinez-Inigo, et al., Biodegradability of extractives in sapwood and heartwood from scots pine by sapstain and white rot fungi, Holzforschung 1999, 53(3), 247-252; T.A. van Beek, et al., Fungal biotreatment of spruce wood with trametes versicolor for pitch control: Influence on extractive contents, pulping process parameters, paper quality and efluent toxicity, Bioresource Technology 2007, 98, 302-311).

Aus der DE 10 2006 057 566 A1 ist ein Verfahren zur Herstellung eines emissionsreduzierten Rohstoffs bekannt, der aus lignozellulosen Partikeln oder deren chemischen Hauptbestandteilen besteht oder diese als Komponente enthält. Es wird eine enzymatisch katalysierte Reaktion ggf. in Anwesenheit von Tensiden durchgeführt; wobei geruchs- und emissionsrelevante Stoffe der Rohstoffpartikel vernetzt oder abgebaut werden. Durch eine anschließende Entwässerung der Suspension werden niedermolekulare Bestandteile sowie die Enzyme wieder entzogen.

Aus der EP 2 138 528 A1 ist ein Verfahren zur Herstellung eines Materials auf Basis von Cellulose, insbesondere von Holz und/oder einem Holzwerkstoff, mit einem verringerten Gehalt an Holzextraktstoff und/oder VOC bekannt, in dem das Material auf Basis von Cellulose mit einem Oxidationskatalysator, insbesondere mit dem Enzym Laccase, behandelt wird, um eine autokatalytische Oxidationsreaktion zu initiieren.

Ein Nachteil an der Verwendung von gereinigten Enzymen, wie beispielsweise eines Laccase-Mediator-Systems, besteht darin, dass diese meist nicht nur Holzextraktstoff, sondern auch Lignin oxidieren. So werden Laccase-Mediator-Systeme beispielsweise auch für die Bleichung von Zellstoff verwendet. Diese Eigenschaft der gereinigten Laccase-Mediator-Systeme kann bei ihrer Verwendung zu unerwünschten Oxidationsreaktionen führen und die Stabilität der behandelten Substrate beeinträchtigen.

Die bisher aus dem Stand der Technik bekannten Verfahren sind insbesondere zur Verringerung des gesamten Spektrums an VOCs in Holzwerkstoffen unzureichend.

Der Erfindung liegt die Aufgabe zugrunde, ein effektives, kostengünstiges und nachhaltiges Verfahren bereitzustellen, mit dem der Gehalt an Holzextraktstoff, Vorläufersubstanzen von VOC und/oder VOC in einem Material auf Basis von Cellulose, insbesondere Holz und/oder einem Holzwerkstoff, verringert werden kann.

Diese Aufgabe wird erfindungsgemäß durch ein Verfahren zur Herstellung eines Holzwerkstoffs nach Anspruch 1 gelöst, wonach ein Holz enthaltendes Vor- oder Zwischenprodukt des Holzwerkstoffs mit einem Mikroorganismen enthaltenden Medium behandelt wird. Ferner offenbart sind ein Holzwerkstoff, ein Mikroorganismen enthaltendes Medium zur Verringerung des Gehalts an Holzextraktstoff, sowie die Verwendung eines Mikroorganismen enthaltenden Mediums zur Verringerung des Gehalts an Holzextraktstoff, Vorläufersubstanzen von VOC und/oder VOC in Holz oder einem Holzwerkstoff gemäß Anspruch 19. Vorteilhafte Ausgestaltungen der Erfindung sind in den abhängigen Ansprüchen angegeben und werden nachfolgend wie der allgemeine Erfindungsgedanke im Einzelnen erläutert.

Das erfindungsgemäße Verfahren zur Herstellung eines Holzwerkstoffs ist dadurch gekennzeichnet, dass ein Holz enthaltendes Vor- oder Zwischenprodukt des Holzwerkstoffs mit einem Mikroorganismen enthaltenden Medium behandelt wird.

Unter "Mikroorganismus" im Sinne der Erfindung wird jede Art von mikroskopisch kleinem Lebewesen verstanden. Mikroskopisch klein bedeutet dabei, dass das Einzellebewesen mit dem bloßen Auge nicht mehr sichtbar ist. Dies ist in der Regel bei Einzellebewesen mit Durchmessern von 100 $\mu$m und kleiner der Fall. Der Begriff "Mikroorganismus", wie hier verwendet, umfasst sowohl pro- als auch eukaryontische Lebewesen und neben einzelligen Lebewesen, wie beispielsweise Bakterien, Hefen, Geißel- oder Wimpertiere, auch mehrzellige Lebewesen, wie z.B. Rädertiere. Zu Mikroorganismen im Sinne der Erfindung gehören insbesondere solche, die ausgewählt sind aus der Gruppe bestehend aus Bakterien, Archaea, Pilzen, Mikroalgen und Protozoen.

Wenn hier von "Mikroorganismen" im Plural die Rede ist, so ist damit eine Pluralität (mindestens zwei) von unterschiedlichen Mikroorganismenarten und nicht etwa nur eine Vielzahl von Einzellebewesen einer Mikroorganismenart gemeint.

Unter dem Begriff "Holzwerkstoff" werden erfindungsgemäß verschiedenste Werkstoffe verstanden, die aus Holz bestehen oder Holz enthalten. Beispiele für Holzwerkstoffe im Sinne der Erfindung sind Holzplatten oder Holzformteile, wobei diese sowohl Verbundwerkstoffe aus einzelnen Holzpartikeln als auch Werkstoffe aus Massivholz sein können. Besonders gute Ergebnisse stellen sich bei dem erfindungsgemäßen Verfahren ein, wenn der Holzwerkstoff ein Holzverbundwerkstoff ist, der durch Zerkleinerung von Cellulose enthaltenden Materialien und/oder anschließendem Zusammenfügen der Strukturelemente erzeugt wird. Solche Strukturelemente können jegliche Zerkleinerungprodukte von Cellulose enthaltenden Materialien sein, wie beispielsweise Holzpartikel, insbesondere Holzspäne, Holzstrands, Holzfasern und/oder Holzfurniere. Holzwerkstoffe im Sinne dieser Erfindung sind insbesondere solche auf Vollholzbasis, Furnierwerkstoffe, Spanwerkstoffe, Faserwerkstoffe oder andere Verbundwerkstoffe. Dabei eignet sich das erfindungsgemäße Verfahren besonders gut zur Herstellung von gepressten Holzwerkstoffen, insbesondere von Faser- oder OSB-Platten.

Je nach Holzwerkstoff kann das Holz enthaltende Vor- oder Zwischenprodukt, welches im erfindungsgemäßen Verfahren mit einem Mikroorganismen enthaltenden Medium behandelt wird, unterschiedlich sein. Wird der Holzwerkstoff durch Zerkleinern von Cellulose enthaltenden Materialien und anschließendes Zusammenfügen der Strukturelemente erzeugt, so kann das Vor- oder Zwischenprodukt beispielsweise ausgewählt sein aus 1.) dem Cellulose enthaltenden Ausgangsmaterial; 2.) den durch Zerkleinerung erhaltenen Strukturelementen, wie beispielsweise den Holzpartikeln, Holzspänen, Holzstrands, Holzfasern oder Holzfurnieren und 3.) dem fast fertigen Holzwerkstoff.

Grundsätzlich ist die Reaktionsgeschwindigkeit des erfindungsgemäßen Verfahrens umso höher, je größer die Oberfläche des zu behandelnden Vor- oder Zwischenproduktes des Holzwerkstoffs ist. Die Oberfläche nimmt bekanntermaßen mit abnehmender Partikelgröße zu. Somit wird im erfindungsgemäßen Verfahren eine höhere Reaktionsgeschwindigkeit erzielt, wenn das zu behandelnde Vor- oder Zwischenprodukte des Holzwerkstoffs eine kleine Partikelgröße aufweist.

Besonders gute Ergebnisse stellen sich ein, wenn das zu behandelnde Vor- oder Zwischenprodukte des Holzwerkstoffs Holzpartikel (insbesondere Holzspäne, Holzstrands oder Holzfasern) sind, deren maximaler Durchmesser 2 cm oder kleiner, bevorzugt 1,5 cm oder kleiner beträgt.

Im Unterschied zu den bekannten Verfahren ist das erfindungsgemäße Verfahren jedoch nicht darauf beschränkt, kleinstückige Materialien, wie beispielsweise Späne, Strands oder Fasern, als Substrate einzusetzen. Vielmehr kann es ausreichen, lediglich die Oberfläche oder lediglich einen Teil der Oberfläche des zu behandelnden Materials mit einem Mikroorganismen enthaltenden Medium zu behandeln, um eine Reduzierung der VOC oder VOC-Vorläufersubstanzen für das gesamte Material zu bewirken.

So kann im Sinne dieser Erfindung auch die Oberfläche eines fast fertigen Holzwerkstoffs, beispielsweise einer gepressten Span-, Faser- oder OSB-Platte, mit einem Mikroorganismen enthaltenden Medium behandelt werden, um damit den erfindungsgemäßen Effekt einer Reduzierung der VOC-Emissionen im Endprodukt zu erzielen.

Überraschend wurde gefunden, dass der Gehalt an Holzextraktstoff, Vorläufersubstanzen von VOC und/oder VOC in Materialien auf Basis von Cellulose, wie beispielsweise Holz oder einem Holzwerkstoff, auf besonders einfache und kostengünstige Weise verringert werden kann, wenn das Material auf Basis von Cellulose oder ein Vor- oder Zwischenprodukt davon mit einem Mikroorganismen enthaltenden Medium behandelt wird.

Dabei wurde festgestellt, dass die unterschiedlichsten Mikroorganismen enthaltenden Medien den erfindungsgemäßen Effekt einer Reduzierung der VOC-Emissionen im Endprodukt bewirken können.

Ein besonderer Vorteil des erfindungsgemäßen Verfahrens besteht darin, dass durch Behandlung mit einem Mikroorganismen enthaltenden Medium nicht nur einzelne VOC-Spezies selektiv reduziert werden, sondern der Gesamt VOC-Gehalt über das gesamte VOC-Spektrum reduziert werden kann. Ohne an wissenschaftliche Theorien gebunden sein zu wollen, scheint dieser Effekt auf dem komplexen Zusammenspiel der verschiedenen Mikroorganismenarten, die in dem Mikroorganismen enthaltenden Medium vorkommen, zu beruhen.

Mit dem erfindungsgemäßen Verfahren werden die unterschiedlichen Stoffwechselleistungen verschiedenster Mikroorganismen in Synergie genutzt. Dabei können Stoffe, die ein Einzelorganismus alleine nicht metabolisieren kann, in Zusammenarbeit unterschiedlich wirkender Mikroorganismen im Idealfall zu Wasser und Kohlendioxid umgesetzt werden.

Gemäß einer bevorzugten Ausführungsform enthält das erfindungsgemäße Medium eine Vielzahl unterschiedlicher Mikroorganismenarten, also mindestens zwei Mikroorganismenarten, bevorzugt mindestens 10, mindestens 100, mindestens 250 oder mindestens 500 verschiedene Mikroorganismenarten. Es wurde beobachtet, dass ein möglichst großer Artenreichtum in dem Mikroorganismen enthaltenden Medium zu einer besonders hohen Leistungsfähigkeit in Bezug auf die VOC-Reduktion führt. Insbesondere wird durch den Artenreichtum das Spektrum an VOC-Spezies und entsprechenden VOC-Vorläufersubstanzen erweitert, welches durch das erfindungsgemäße Verfahren reduziert werden kann.

Eine Vielzahl von unterschiedlichen Mikroorganismen, die zusammen in einem Medium oder Habitat leben, findet sich in der Natur häufig in Form von sogenannten Mikrobiozönosen. Gemäß einer bevorzugten Ausführungsform der Erfindung stammen die in dem erfindungsgemäßen Medium enhaltenen Mikroorganismen aus einer Mikrobiozönose oder stellen eine Mikrobiozönose dar.

Unter einer Mikrobiozönose versteht der Fachmann ein Konsortium von Mikroorganismen oder eine Mikroorganismengemeinschaft, welche die Gesamtheit der in einem Mikrohabitat vorkommenden Mikroorganismen darstellt. Mikrobiozönosen stellen ein dynamisches System dar, das sich beständig ändert und sich laufend an die Umgebungs- und Nährstoffbedingungen anpasst. Bleiben Individuen- und Artenzahlen über einen längeren Zeitraum konstant, spricht man davon, dass sich die Mikrobiozönose im Gleichgewicht befindet. Die ständige Änderung der Zusammensetzung an Mikroorganismen und die Anpassungsfähigkeit von Mikrobiozönosen im Allgemeinen erklärt vermutlich auch, warum gefunden wurde, dass in dem erfindungsgemäßen Verfahren eine Vielzahl von in der Natur vorkommenden Mikrobiozönosen erfolgreich eingesetzt werden kann. Dies gilt selbst für den Fall, dass die Mikrobiozönosen nicht durch ihre ursprüngliche, natürliche Umgebung an die Verstoffwechselung von Holzextraktstoffen, VOC-Vorläufersubstanzen oder VOC gewöhnt waren. Vermutlich führt in solchen Fällen eine rasche Adaption der Mikrobiozönose an die zu behandelnden Vor- oder Zwischenprodukte des Holzwerkstoffs als neue Nährstoffquelle zu der beobachteten erfindungsgemäßen Verringerung des Gehalts an Holzextraktstoff, Vorläufersubstanzen von VOC und/oder VOC.

Der Erfolg des erfindungsgemäß eingesetzten Mikroorganismen enthaltenden Mediums beruht vermutlich darauf, dass die Nährstoffansprüche und Stoffwechselwege der einzelnen darin enthaltenen Mikroorganismenarten sehr unterschiedlich sind und dadurch beispielsweise das Stoffwechselprodukt des einen Mikroorganismus das Substrat für einen anderen Mikroorganismus darstellen kann. Hierdurch lässt sich vermutlich erklären, warum sich mit dem erfindungsgemäßen Verfahren der Gesamtgehalt an VOC über das gesamte VOC-Spektrum verringern lässt und nicht nur einzelne VOC-Spezies selektiv reduziert werden.

Das erfindungsgemäß einzusetzende Medium kann die Mikroorganismen planktonisch (d.h. wie Plankton in Wasser schwebend), flokkuliert (d.h. als Aneinanderballung kleinerer Gruppen von Mikroorganismen) oder in Form eines intakten oder zerkleinerten Biofilms enthalten.

Wenn hier von Medium die Rede ist, so ist damit jede Flüssigkeit gemeint, in der die eingesetzten Mikroorganismen überleben können. Gemäß einer bevorzugten Ausführungsform wird als Medium eine Flüssigkeit gewählt, in der die eingesetzten Mikroorganismen sich vermehren und/oder stoffwechselaktiv sein können. Vorzugsweise ist das Mikroorganismen enthaltende Medium ein Medium auf Basis von Wasser, d.h. der Wasseranteil in der flüssigen Phase liegt bei mindestens 50 Gew.-%, vorzugsweise mindestens 80 Gew.-% und insbesondere bevorzugt mindestens 90 Gew.-% bzw. mindestens 95 Gew.-% bezogen auf das Gesamtgewicht der flüssigen Bestandteile des Mediums. Gemäß einer weiteren Ausführungsform der Erfindung wird als Medium Wasser eingesetzt, in welches die Mikroorganismen eingebracht werden. Das Mikroorganismen enthaltende Medium kann auch eine Mikroorganismenkultur oder eine Verdünnung hiervon sein.

Gemäß einer weiteren Ausführungsform der Erfindung stammen die in dem erfindungsgemäßen Medium enthaltenen Mikroorganismen aus einem Biofilm. Unter einem Biofilm versteht der Fachmann eine spezielle Form einer Mikrobiozönose, in der die verschiedenen Mikroorganismenarten, ggf. zusammen mit anderen partikulären Stoffen wie z.B. Sand, sich an einer Oberfläche oder Grenzfläche angelagert haben. Biofilme enthalten meist eine Schleimmatrix aus extrazellulären polymeren Substanzen (EPS), in denen die Zellen immobilisiert sind. Die Voraussetzungen für die Entstehung von Biofilmen - nämlich das Zusammentreffen von Mikroorganismen, Grenzflächen, Nährstoffen und Feuchtigkeit - sind fast überall gegeben. Biofilme stellen für Mikroorganismen einen Schutz- und Lebensraum dar, der ein symbiotisches Zusammenleben verschiedenster Arten begünstigt. Vorteilhaft gegenüber einer planktonischen Lebensweise (als Einzelorganismus frei schwebend oder schwimmend) ist bei Biofilmen vor allem der Schutz vor widrigern Umwelteinflüssen, wie beispielsweise Austrocknung, extreme pH-Werte oder Chemikalien und Desinfektionsmitteln. Zumindest die in tiefer liegenden Schichten des Biofilms lebenden Mikroorganismen sind vor solchen Umwelteinflüssen geschützt.

Dies erklärt vermutlich auch, warum sich in dem Schutzraum eines Biofilms die verschiedensten Mikroogranismenarten optimal entfalten können. Dieser besondere Artenreichtum wird insbesondere auch durch das Vorhandensein ökologischer Nischen im Biofilm (z.B. Strömungs-Totzonen oder sauerstofffreie Zonen in den tieferen Schichten des Biofilms) weiter unterstützt. Hinzu kommt, dass die EPS-Matrix auch als Nährstoffspeicher für schlechte Zeiten fungiert.

Biofilme, die für das erfindungsgemäße Verfahren besonders geeignete Mikroorganismen enthalten, sind weit verbreitet. So bilden sich geeignete Biofilme beispielsweise an der Oberfläche und an den Wand- und Bodenflächen von stehenden Wässern, wie beispielsweise in Abwasserbehältern oder Regenauffangbecken. Erfindungsgemäß bevorzugt sind Biofilme, die aus Abwasser, der biologischen Abwasseraufbereitung oder Anlagen der Holz- und/oder holzverarbeitenden Industrie stammen und/oder Biofilme, die schon mit Holz und/oder Holzbestandteilen in Berührung gekommen sind. Bevorzugte Abwasserarten, in denen für das erfindungsgemäße Verfahren geeignete Biofilme mit Mikroorganismen vorhanden sind, sind Abwasserarten aus der Holzwerkstoffproduktion, insbesondere Abwasser, das aus der Aufbereitung von Holz durch Dämpfen, Kochen, Zerfasern oder Trocknen, stammt.

Beispielsweise kann als Abwasser aus der Holzwerkstoffproduktion Stopfschneckenaustragswasser eines Sägespan-Refiners bzw. eines Hackschnitzel-Refiners verwendet werden. Ein Refiner dient der Zerfaserung von Hackschnitzeln oder Sägespänen. Die Stopfschnecke ist dem Refiner vorgeschaltet und dient zur Entwässerung und Zuleitung zum Refiner. Die Hackschnitzel oder Sägespäne werden vor dem Refiner gekocht um das Material aufzuweichen. Das überschüssige Wasser inklusive der extrahierten Holzbegleitstoffe wird dann in der Stopfschnecke vor dem Refiner abgetrennt, welches dann für diese Anwendung zur Verfügung steht. Das Stopfschneckenaustragswasser ist zwar aufgrund der im Kocher herrschenden Bedingungen (bis zu 8 bar Druck und 160°C) zunächst annähernd steril, erfährt jedoch im nachgeschalteten Sammeltank nach dem Abkühlen eine Besiedelung durch Mikroorganismen.

Die mit dem erfindungsgemäßen Verfahren behandelten Materialien auf Basis von Cellulose, wie beispielsweise die Holz enthaltenden Vor- oder Zwischenprodukte eines Holzwerkstoffs, weisen einen verringerten Gehalt an Holzextraktstoff, Vorläufersubstanzen von VOC und/oder VOC auf. Insbesondere ermöglicht das erfindungsgemäße Verfahren eine wirkungsvolle Verringerung des Gehalts an ungesättigten Verbindungen im Holzextraktstoff wie beispielsweise ungesättigte Fettsäuren oder ungesättigte Fettsäureester, z.B. Linolsäure oder Triglyceride mit mindestens einem ungesättigten Fettsäurebestandteil. Da diese Verbindungen VOC-Vorläufersubstanzen für die VOC-Klasse der Aldehyde darstellen, lassen sich mit dem erfindungsgemäßen Verfahren Aldehydemissionen besonders wirkungsvoll verringern.

Unter dem Begriff Holzextraktstoff werden erfindungsgemäß ein/oder mehrere Holzinhaltsstoffe verstanden, die mit polaren und/oder apolaren organischen Lösungsmitteln und/oder Wasser, beispielsweise mit Wasser, Alkohol, Benzol, Hexan, Cyclohexan, Ether und/oder Aceton aus dem Holz extrahiert werden können.

Wie bereits diskutiert entstehen zusätzlich zu den schon vorhandenen VOCs aus den im Holzextraktstoff enthaltenen VOC-Vorläuferverbindungen in der Wärme weitere VOC-Spezies. Vermutlich aufgrund des verringerten Gehalts an Holzextraktstoff und VOC-Vorläuferverbindungen zeichnet sich das mit dem erfindungsgemäßen Verfahren behandelte Vor- oder Zwischenprodukt eines Holzwerkstoffs auch dadurch aus, dass bei seiner Erwärmung, beispielsweise in einem Trocknungsschritt, eine geringere Menge VOC freigesetzt wird. Gleiches gilt für das Endprodukt, den Holzwerkstoff, der ebenfalls geringere VOC-Emissionswerte aufweist.

VOCs, deren Gehalt mit dem erfindungsgemäßen Verfahren verringert werden soll, sind beispielsweise aromatische Kohlenwasserstoffe, aliphatische Kohlenwasserstoffe, Terpene, aliphatische Alkohole, aromatische Alkohole, Glykole, Glykolether, Glykolester, Aldehyde, Ketone, organische Säuren, Ester und Lactone.

Zu den VOCs, deren Gehalt mit dem erfindungsgemäßen Verfahren verringert werden soll, zählen beispielsweise auch aromatische Kohlenwasserstoffe, wie beispielsweise Toluol, Ethylbenzol, o-, m- und p-Xylol, Isopropylbenzol, n-Propylbenzol, 1-Propenylbenzol, 1,3,5-Trimethylbenzol, 1,3,5-Trimethylbenzol, 1,2,3-Trimethylbenzol, 2-Ethyltoluol, 1-Isopropyl-2-methylbenzol (o-Cymol), 1-Isopropyl-3-methylbenzol (m-Cymol), 1-Isopropyl-4-methylbenzol (p-cymol), 1,2,4,5-Tetramethylbenzol, 1,2,4,5-Tetramethylbenzol, 1,2,4,5-Tetramethylbenzol, 1,4-Diisopropylbenzol, 1,4-Diisopropylbenzol, 1-Phenyldecan und Isomere, 1-Phenylundecan und Isomere, 4-Phenylcyclohexen (4-PCH), Styrol, Phenylacetylen, 2-Phenylpropen (a-Methylstyrol), Vinyltoluol (alle Isomeren: o-, m-, p-Methylstyrole), Alkylbenzole, Naphthalin oder Inden.

Ferner sind von den VOCs, deren Gehalt mit dem erfindungsgemäßen Verfahren verringert werden soll, beispielsweise auch aliphatische Kohlenwasserstoffe, wie zum Beispiel 3-Methylpentan, n-Hexan, Cyclohexan, Methylcyclohexan, n-Heptan, sowie andere gesättigte aliphatische Kohlenwasserstoffe mit 6 bis 8 oder 9 bis 16 Kohlenstoffatomen, umfasst.

Eine wichtige Untergruppe der VOCs, deren Gehalt mit dem erfindungsgemäßen Verfahren verringert werden soll, sind beispielsweise Terpene, wie zum Beispiel 3-Caren, α-Pinen, ß-Pinen, Limonen und sonstige Terpene.

Zu den VOCs, deren Gehalt mit dem erfindungsgemäßen Verfahren verringert werden soll, zählen beispielsweise des Weiteren aliphatische Alkohole, wie zum Beispiel Ethanol, 1-Propanol, 2-Propanol, tert-Butanol, 2-Methyl-2-propanol, 2-Methyl-1-propanol, 1-Butanol, Pentanol (alle Isomere), 1-Hexanol, Cyclohexanol, 2-Ethyl-1-hexanol, 1-Octanol, 4-Hydroxy-4-methyl-pentan-2-on (Diacetonalkohol), andere C4-C10 gesättigte n- und iso- Alkohole, andere C11-C13 gesättigte n- oder iso-Alkohole sowie aromatische Alkohole, wie beispielsweise Phenol, BHT (2,6-Di-tert-butyl-4-methylphenol), Benzylalkohol.

Eine weitere Untergruppe der VOCs, deren Gehalt mit dem erfindungsgemäßen Verfahren verringert werden soll, sind beispielsweise Glykole, Glykolether und Glykolester, zum Beispiel Propylenglykol (1,2-Dihydroxypropan), Ethylenglykol (Ethandiol), Ethylenglykolmonobutylether, Diethylenglykol, Diethylenglykolmonobutylether, 2-Phenoxyethanol, Ethylencarbonat, 1-Methoxy-2-propanol, 2,2,4-Trimethyl-1,3-pentandiolmonoisobutyrat (Texanol®), Glykolsäurebutylester (Hydroxyessigsäurebutylester), Butyldiglykolacetat (Ethanol, 2-(2-butoxyethoxy)acetat, BDGA), Dipropylenglykolmonomethylether, 2-Methoxyethanol, 2-Ethoxyethanol, 2-Propoxyethanol, 2-Methylethoxyethanol, 2-Hexoxyethanol, 1,2-Dimethoxyethan, 1,2-Diethoxyethan, 2-Methoxyethylacetat, 2-Ethoxyethylacetat, 2-Butoxyethylacetat, 2-(2-Hexoxyethoxy)-ethanol, 1-Methoxy-2-(2-methoxyethoxy)-ethan, 2-Methoxy-1-propanol, 2-Methoxy-1-propylacetat, Propylenglykoldiacetat, Dipropylenglykol, Dipropylenglykolmonomethyletheracetat, Dipropylenglykolmononpropylether, Dipropylenglykolmono-nbutylether, Dipropylenglykolmono-tbutylether, 1,4-Butandiol, Tripropylenglykolmonomethylether, Triethylenglykoldimethylether, 1,2-Propylenglykoldimethylether, TXIB, Ethyldiglykol, Dipropylenglykoldimethylether, Propylencarbonat, Hexylenglykol (2-Methyl-2,4-pentandiol), 3-Methoxy-1-butanol, 1,2-Propylenglykol-npropylether, 1,2-Propylenglykolnbutylether, Diethylenglykol-phenylether und Neopentylglykol (2,2-Dimethylpropan-1,3-diol).

Ferner sind von den VOCs, deren Gehalt mit dem erfindungsgemäßen Verfahren verringert werden soll, beispielsweise auch Aldehyde, wie zum Beispiel Butanal, Pentanal, Hexanal, Heptanal, Octanal, Nonanal, Decanal, 2-Butenal (Crotonaldehyd, cistrans-Gemisch), 2-Pentenal, 2-Hexenal, 2-Heptenal, 2-Octenal, 2-Nonenal, 2-Decenal, 2-Undecenal, Furfural, Glutaraldehyd, Benzaldehyd, Acetaldehyd oder Propanal, sowie Ketone, wie beispielsweise Ethylmethylketon, 3-Methyl-2-butanon, Methylisobutylketon, Cyclopentanon, Cyclohexanon, 2-Methylcyclopentanon, 2-Methylcyclohexanon, Acetophenon, 1-Hydroxyaceton (1-Hydroxy-2-propanon) oder Aceton, umfasst.

Eine weitere Untergruppe der VOCs, deren Gehalt mit dem erfindungsgemäßen Verfahren verringert werden soll, sind organische Säuren, wie beispielweise Essigsäure, Propionsäure, Isobuttersäure, Buttersäure, Pivalinsäure, n-Valeriansäure, n-Capronsäure, n-Heptansäure, n-Octansäure oder 2-Ethylhexansäure.

Beispiele für Ester und Lactone, die zu den VOCs gehören, deren Gehalt mit dem erfindungsgemäßen Verfahren verringert werden soll, sind beispielsweise Methylacetat, Ethylacetat, Vinylacetat, Isopropylacetat, Propylacetat, 2-Methoxy-1-methylethylacetat, n-Butylformiat, Methylmethacrylate, Isobutylacetat, 1-Butylacetat, 2-Ethylhexylacetat, Methylacrylat, Ethylacrylat, n-Butylacrylat, 2-Ethylhexylacrylat, Acrylsäureester, Adipinsäuredimethylester, Fumarsäuredibutylester, Bernsteinsäuredimethylester, Glutarsäuredimethylester, Hexandioldiacrylat, Maleinsäuredibutylester, Butyrolacton, Glutarsäurediisobutylester, Bernsteinsäurediisobutylester.

Ferner gehören auch folgende Verbindungen zu den VOCs, deren Gehalt mit dem erfindungsgemäßen Verfahren verringert werden soll: Beispielsweise 1,4-Dioxan, Caprolactam, N-Methyl-2-pyrrolidon, Octamethylcyclotetrasiloxan, Methenamin, Hexamethylentetramin (Formaldehydabspalter), 2-Butanonoxim, Tributylphosphat, Triethylphosphat, 5-Chlor-2-methyl-4isothiazolin-3-on (CIT), 2-Methyl-4-isothiazolin-3-on (MIT), Triethylamin, Decamethylcyclopentasiloxan, Dodecamethylcyclohexasiloxan, Tetrahydrofuran und Dimethylformamid.

Als besonders wirkungsvoll hat sich das erfindungsgemäße Verfahren bei der Reduktion des Gehalts an VOCs der Aldehydklasse und Vorläuferverbindungen der Aldehydemissionen (Fettsäuren) erwiesen.

Das erfindungsgemäße Verfahren zur Herstellung eines Holzwerkstoffs beinhaltet den Schritt, dass ein Holz enthaltendes Vor- oder Zwischenprodukt des Holzwerkstoffs mit einem Mikroorganismen enthaltenden Medium behandelt wird.

Wenn hier von "Behandlung" die Rede ist, so ist damit eine aktive Handlung gemeint. Insbesondere ist damit das in Berührung bringen des Holz enthaltenden Vor- oder Zwischenprodukts mit dem Mikroorganismen enthaltenden Medium und ggf. eine sich anschließende Inkubation gemeint. Nicht von dem Begriff der "Behandlung", wie hier verwendet, umfasst sind Prozesse in denen Mikroorganismen, die schon von vorneherein auf oder in dem Holz enthaltenden Vor- oder Zwischenprodukte des Holzwerkstoffs vorhanden waren, auf diesen im Laufe der Zeit einwirken und ihn zu zersetzen beginnen (beispielsweise während der Lagerung, Verarbeitung oder Kompostierung). Der Begriff der "Behandlung", wie hier verwendet, enthält zwingend den Schritt, dass Mikroorganismen dem Holz enthaltenden Vor- oder Zwischenprodukt des Holzwerkstoffs aktiv zugeführt werden, bzw. mit diesem in Berührung gebracht werden.

Gemäß einer bevorzugten Ausführungsform der Erfindung wird das Holz enthaltende Vor- oder Zwischenprodukt des Holzwerkstoffs behandelt, indem es mit dem Mikroorganismen enthaltenden Medium benetzt wird. Vorzugsweise wird das benetzte Vor- oder Zwischenprodukt des Holzwerkstoffs anschließend inkubiert.

Unter "Benetzen" im Sinne der Erfindung wird jedes in Berührung bringen des Holz enthaltenden Vor- oder Zwischenprodukts mit einem Mikroorganismen enthaltenden Medium verstanden, insbesondere das Auftragen dieses Mediums auf die Oberfläche des Vor- oder Zwischenprodukts. Es hat sich als besonders vorteilhaft erwiesen, wenn das Verhältnis Flüssigkeit (Mikroorganismen enthaltendes Medium) zu dem Vor- oder Zwischenprodukts dabei so ist, dass das Vor- oder Zwischenprodukt nicht in dem Medium schwimmt bzw. suspendiert ist, sondern lediglich von diesem befeuchtet wird. Diese Bedingung ist in aller Regel dann erfüllt, wenn die Holzfeuchte insgesamt, also inklusive des zur Benetzung verwendeten Mikroorganismen enthaltenden Mediums, höchstens etwa 300 %, bevorzugt höchsten etwa 250 % und besonders bevorzugt von etwa 130 % bis etwa 250 %, beträgt. Die Oberfläche des Holz enthaltenden Vor- oder Zwischenprodukts kann vollständig oder nur teilweise mit Mikroorganismen enthaltenden Medium benetzt sein.

Gemäß einer bevorzugten Ausführungsform der Erfindung weist das Holz enthaltende Vor- oder Zwischenprodukt des Holzwerkstoffs bei der Behandlung mit dem Mikroorganismen enthaltenden Medium eine Holzfeuchte von höchstens etwa 300 %, bevorzugt höchstens etwa 250 % und besonders bevorzugt von etwa 130 % bis etwa 250%, auf.

Es wurde festgestellt, dass sich bei diesen Holzfeuchten optimale Ergebnisse in Bezug auf VOC-Reduktion sowie die mechanisch-chemischen Eigenschaften der hergestellten Holzwerkstoffe ergeben. Dabei bedeutet "Holzfeuchte" erfindungsgemäß den Feuchtegehalt des Holzes, d.h. das Verhältnis der im Holz enthaltenen und ggf. das Holz umgebenden Wassermenge zum Holztrockengewicht (Darrgewicht) des Holzes in Gewichtsprozent. Die Holzfeuchte lässt sich erfindungsgemäß nach DIN EN 322 unter Anwendung folgender Formel berechnen: Holzfeuchte (%) = 100 x (Nassgewicht - Darrgewicht) / Darrgewicht. Dabei bedeutet Darrgewicht wie hier verwendet das Gewicht nach Trocknung bei einer Temperatur von 103°C ± 2°C bis zur Massekonstanz, wie in DIN EN 322 beschrieben.

Da das erfindungsgemäße Verfahren schon mit geringen Holzfeuchten während der Behandlung mit dem Mikroorganismen enthaltenden Medium sehr gute Ergebnisse liefert, ist es möglich, den Wasserverbrauch und den trocknungsbedingten Energieverbrauch in der Produktion erheblich zu reduzieren. Dies ist im Hinblick auf die steigenden Energiekosten von Vorteil und entspricht den Anforderungen an eine nachhaltige und umweltschonende Produktionsweise.

Grundsätzlich sind dem Fachmann vielfältige Möglichkeiten zur Benetzung und/oder zum Einstellen der gewünschten Holzfeuchte bekannt. Als besonders praxisgerecht für das erfindungsgemäße Verfahren hat sich eine Benetzung durch Sprühen, Bedüsen, Spritzen und/oder Benebeln erwiesen. Es ist aber auch denkbar, dass das Holz enthaltende Vor- oder Zwischenprodukt beispielsweise in ein Flüssigkeitsbad, welches mit dem Mikroorganismen enthaltenden Medium befüllt ist, eingetaucht und anschließend wieder entnommen wird, um eine Benetzung zu bewirken. Auch ein Tränken oder Fluten des Holz enthaltenden Vor- oder Zwischenprodukts ist denkbar.

Vorzugsweise umfasst die Behandlung des Vor- oder Zwischenprodukts des Holzwerkstoffs mit dem Mikroorganismen enthaltenden Medium auch einen Inkubationsschritt. Dabei wird das Gemisch aus Holz enthaltendem Vor- oder Zwischenprodukt und Mikroorganismen enthaltendem Medium vorzugsweise bei etwa 15°C bis etwa 70°C inkubiert. Gemäß einer bevorzugten Ausführungsform des erfindungsgemäßen Verfahrens erfolgt die Inkubation bei etwa 15°C bis etwa 60°C, bevorzugt bei etwa 15°C bis etwa 50°C und insbesondere bei etwa 15°C bis etwa 40°C. Es sind damit keine Vorkehrungen zur Temperierung während der Inkubation erforderlich und diese kann insbesondere auch bei Raumtemperatur erfolgen. Entsprechend kann, wenn es sich bei dem hergestellten Holzwerkstoff um einen gepressten Holzwerkstoff, wie beispielsweise eine Faser- oder OSB-Platte, handelt, der Inkubations- und/oder Behandlungsschritt in einem herkömmlichen Naßspanbunker vor der Trocknung bzw. vor dem Kocher erfolgen.

Da die erfindungsgemäße Behandlung mit dem Mikroorganismen enthaltenden Medium schon bei geringen Temperaturen - und insbesondere auch bei Raumtemperatur - sehr gute Ergebnisse liefert, ist es möglich, den Energieverbrauch in der Holzwerkstoffproduktion erheblich zu reduzieren. Dies ist im Hinblick auf die Verfahrensökonomie und die Energieeffizienz des erfindungsgemäßen Verfahrens vorteilhaft. Die Holzpartikel können beispielsweise nach dem in Berührung bringen mit dem Mikroorganismen enthaltenden Medium einfach in dem zur Applikation des Mediums verwendeten Apparat gelassen werden, um dort bei Raumtemperatur inkubiert zu werden. Versuche haben ergeben, dass das erfindungsgemäße Verfahren bei allen, je nach geographischer Lage und Jahreszeit, herrschenden Umgebungstemperaturen, insbesondere zwischen etwa 15°C und etwa 40°C, sehr gute Ergebnisse liefert.

In Bezug auf die bevorzugte Inkubationsdauer für die Behandlung mit dem Mikroorganismen enthaltenden Medium werden optimale Ergebnisse erzielt, wenn die Behandlung oder die Inkubation für mindestens etwa 180 Minuten erfolgt. Ab dieser Behandlungs- bzw. Inkubationsdauer stellt sich eine ausreichende Verringerung des Gehalts an Holzextraktstoff, Vorläufersubstanzen von VOC und/oder VOC ein. Eine längere Inkubationsdauer ist in der Regel vorteilhaft. Je nach Aktivität des Mikroorganismen enthaltenden Mediums, der Beschaffenheit des Holz enthaltenden Vor- oder Zwischenprodukts sowie dessen Gehalt an Holzextraktstoff, Vorläufersubstanzen von VOC und/oder VOC ist beispielsweise auch eine Behandlungs- bzw. Inkubationsdauer von 72 Stunden oder mehr denkbar.

Für viele Anwendungsbereiche hat sich gezeigt, dass das Mikroorganismen enthaltende Medium keinen besonderen pH oder besonderes Puffersystem für den Einsatz im erfindungsgemäßen Verfahren aufweisen muss. Mithin ist ein weiterer Vorteil des erfindungsgemäßen Verfahrens darin zu sehen, dass sich vielfach gute Ergebnisse erzielen lassen, ohne dass ein bestimmter pH eingestellt oder überwacht werden müsste. Nichtsdestotrotz stellen sich optimale Ergebnisse ein, wenn die Behandlung mit dem Mikroorganismen enthaltenden Medium bei einem pH von etwa 4 bis etwa 6 erfolgt.

In der Praxis hat es sich ferner als vorteilhaft erwiesen, wenn das Gemisch aus Holz enthaltendem Vor- oder Zwischenprodukt und Mikroorganismen enthaltendem Medium zu Beginn und/oder während der Behandlung mechanisch durchmischt wird, um eine möglichst gleichmäßige Verteilung der Mikroorganismen auf dem Holz enthaltenden Vor- oder Zwischenprodukt zu gewährleisten. Zum Mischen kann beispielsweise ein Pflugscharmischer eingesetzt werden. Dem Fachmann sind aber - abhängig von Menge und Größe des Holz enthaltenden Vor- oder Zwischenprodukt - auch andere geeignete Mischapparaturen bekannt. Praktische Versuche haben gezeigt, dass für die meisten Anwendungen ein Durchmischen für etwa 1 bis etwa 10 Minuten, insbesondere für etwa 5 Minuten ausreichend ist, um optimale Ergebnisse zu erzielen.

Mit der erfindungsgemäßen Verfahrensführung ist es möglich, die VOC-Reduktion bei Normaldruck und ohne Anlegen eines Vakuums durchzuführen.

Praktische Versuche haben ferner gezeigt, dass es zur Durchführung des erfindungsgemäßen Verfahrens nicht erforderlich ist, Luft und/oder Sauerstoff während der Behandlung in das Reaktionsgemisch einzubringen. Ebenfalls ist weder Rühren noch Schütteln des Gemischs aus Holz enthaltendem Vor- oder Zwischenprodukt und Mikroorganismen enthaltenden Medium während der Behandlung nötig.

Es kann jedoch zu einer Beschleunigung des erfindungsgemäßen Verfahrens führen, wenn die Behandlung unter aeroben Bedingungen, d.h. in Anwesenheit von Sauerstoff durchgeführt wird bzw. wenn Luft und/oder Sauerstoff während der Behandlung in das Gemisch aus Holz enthaltendem Vor- oder Zwischenprodukt und Mikroorganismen enthaltendem Medium eingebracht wird. Dies gilt insbesondere dann, wenn die in dem erfindungsgemäßen Medium enthaltenen Mikroorganismen auch aerobe Mikroorganismen umfassen. Gemäß einer bevorzugten Ausführungsform der Erfindung wird, wenn die in dem erfindungsgemäßen Medium enthaltenen Mikroorganismen, jeweils bezogen auf die Gesamtmenge an Mikroorganismen Einzellebewesen, mindestens etwa 50 Gew.-%, insbesondere mindestens etwa 70 Gew.-% bzw. mindestens etwa 90 Gew.-% aerobe Mikroorganismen enthalten, die erfindungsgemäße Behandlung des Holz enthaltenden Vor- oder Zwischenprodukts unter Anwesenheit von Luft und/oder Sauerstoff durchgeführt. In diesem Fall ist es vorteilhaft, wenn während der Behandlung eine Belüftung und/oder ein Einbringen von Luft und/oder Sauerstoff in das Gemisch aus Holz enthaltendem Vor- oder Zwischenprodukt und Mikroorganismen enthaltendem Medium erfolgt.

Gemäß einer weiteren möglichen Ausführungsform der Erfindung werden während der Behandlung mit dem Mikroorganismen enthaltenden Medium alternierend aerobe und anaerobe Bedingungen eingestellt, um der Vielfalt der zu verstoffwechselnden Vorläufersubstanzen von VOC und/oder VOC sowie den Ansprüchen der heterogenen natürlichen Mikrobenpopulationen gerecht zu werden.

Gemäß einer bevorzugten Ausführungsform der Erfindung sind die in dem erfindungsgemäßen Medium enthaltenen Mikroorganismen aus Abwasser und/oder Filterrückständen aus der Aufbereitung dieses Abwassers gewonnen worden. Das Mikroorganismen enthaltende Medium kann insbesondere auch Abwasser und/oder Filterrückstände aus der Aufbereitung dieses Abwassers enthalten oder hieraus bestehen. Gemäß einer weiteren Ausführungsform handelt es sich bei den Filterrückständen aus der Aufbereitung des Abwassers um belebte Filterrückstände.

Überraschenderweise hat sich gezeigt, dass es zur Reduktion von Holzextraktstoff, Vorläufersubstanzen von VOC und/oder VOC nicht notwendig ist, besonders ausgewählte Mikroorganismen dem Behandlungsgut zuzusetzen, sondern dass es ausreicht, das Holz enthaltende Vor- oder Zwischenprodukt mit natürlich vorkommenden Mikrobiozönosen, Biofilmen oder sogar mit einem "Abfallprodukt", nämlich dem oben bezeichneten Abwasser und/oder belebten Filterrückständen aus der Aufbereitung von Abwasser, zu behandeln.

Insbesondere Abwasser und/oder Filterrückstände aus der Aufbereitung von Abwasser scheinen nämlich durch das sehr breite Spektrum an in Abwasser vorkommenden Verunreinigungen eine Mikrobiozönose aufzuweisen, die prinzipiell alle zum Abbau von Holzextraktstoff, Vorläufersubstanzen von VOC und/oder VOC benötigten Mikroorganismen enthält. In gängigen Abwassern und/oder belebten Filterrückständen aus der Aufbereitung von Abwassern scheinen also schon spezielle Mikroorganismen enthalten zu sein, die zum Abbau oder zum Kometabolismus von Holzextraktstoff, Vorläufersubstanzen von VOC und/oder VOC fähig sind. Folglich liegen die zur VOC-Reduktion notwendigen Mikroorganismen in gängigen Abwasserarten und/oder belebten Filterrückständen aus der Aufbereitung von Abwasser bereits in geeigneten Mikrobiozönosen und mit Nährstoffen versorgt vor.

Das erfindungsgemäße Verfahren ist vor allem auch aus ökologischer Sicht vorteilhaft, da es eine Möglichkeit bietet, Abwasser und Abfälle (in Form von Filterrückständen) wiederzuverwenden und damit zu recyclen. Die dadurch eingesparten Entsorgungs- und Deponiekosten machen das erfindungsgemäße Verfahren insgesamt auch ökonomisch attraktiv.

Wenn hier von "Abwasser" die Rede ist, so ist damit jedes durch Gebrauch verunreinigte oder von befestigten Flächen abfließende Wasser, das gefasst und/oder abgeleitet wird, gemeint. Diese Definition entspricht der im deutschen Wasserhaushaltsgesetz (WHG v. 31. Juli 2009, BGBl. I S. 2585) verwendeten Definition. "Abwasser" wie hier verwendet bedeutet demnach das durch häuslichen, gewerblichen, landwirtschaftlichen oder sonstigen Gebrauch in seinen Eigenschaften veränderte Wasser und das bei Trockenwetter damit zusammen abfließende Wasser (Schmutzwasser) sowie das von Niederschlägen aus dem Bereich von bebauten oder befestigten Flächen gesammelt abfließende Wasser (Niederschlagswasser). Die aus Anlagen zum Behandeln, Lagern und Ablagern von Abfällen austretenden und gesammelten Flüssigkeiten gelten ebenfalls als Schmutz- und damit als Abwasser.

Der Begriff "Abwasser" wie hier verwendet bedeutet in Anlehnung an die deutsche Trinkwasserverordnung (TrinkwV BGBl I 2001 S. 959 ff.) insbesondere eine Flüssigkeit, welche die Anforderungen an Trinkwasser in Bezug auf die Gesamtkeimzahl nicht mehr erfüllt. Die Gesamtkeimzahl ist eine unspezifische Größe, die durch eine in der TrinkwV beschriebene mikrobiologische Untersuchung bestimmt werden kann. Sie gibt an, wie viele Mikroorganismenkolonien sich auf einem für diesen Zweck normierten Agar-Nährboden im Verlauf von 48 Stunden bei 22 bzw. 36 °C bilden, wenn man 1 ml Wasserprobe auf oder in dem Nährboden verteilt.

Von der nach TrinkwV bestimmten Gesamtkeimzahl werden zwar nicht alle in dem Wasser enthaltenen Mikroorganismen erfasst. Dies ist grundsätzlich unmöglich, wenn das Habitat - wie normalerweise üblich - eine Mikrobiozönose mit breitem Spektrum an physiologisch verschiedenartigen Mikroorganismen enthält. Es gibt nämlich keine Kulturbedingungen (Zusammensetzung des Nährbodens, Inkubationstemperatur und - atmosphäre), unter denen sich alle in einem natürlichen Habitat vorkommenden Mikroorganismen optimal vermehren können. Trotzdem ist die nach TrinkwV bestimmte Gesamtkeimzahl ein guter Indikator für die tatsächlich vorherrschende Gesamtkeimzahl.

Nach der deutschen Trinkwasserverordnung gilt ein Grenzwert von 100 KbE/ml ("Koloniebildende Einheiten je Milliliter") für leitungsgeführtes Trinkwasser aus Brunnen und von 1000 KbE/ml für vorübergehend in Tanks aufbewahrtes Trinkwasser.

Wenn hier also von "Abwasser" die Rede ist, so ist damit insbesondere eine Flüssigkeit gemeint, die eine nach TrinkwV bestimmte Gesamtkeimzahl von mehr als etwa 1000 KbE/ml, insbesondere mehr als etwa 2000, bevorzugt mehr als etwa 3000, mehr als etwa 5000 oder mehr als etwa 10000 KbE/ml aufweist.

Gemäß einer besonderen Ausführungsform der Erfindung ist das Abwasser ausgewählt aus der Gruppe bestehend aus Niederschlagswasser, häusliches Abwasser, kommunales Abwasser, Industrieabwasser, Abwasser von Wasseraufbereitungsanlagen und Kombinationen hiervon.

Praktische Versuche haben gezeigt, dass das erfindungsgemäße Verfahren besonders gut funktioniert, wenn das Abwasser ein Abwasser ist, das mit Holz und/oder Holzbestandteilen in Berührung gekommen ist. Beispiele für solche Abwässer sind Abwässer aus der Holz- und/oder holzverarbeitenden Industrie. Das Abwasser kann beispielsweise aus der Holzwerkstoffproduktion, insbesondere aus der Aufbereitung von Holz durch Dämpfen, Kochen, Zerfasern oder Trocknen, stammen.

Die erfindungsgemäße Lehre kann nach einer weiteren erfindungsgemäßen Ausführungsform auch dadurch verwirklicht werden, dass die in dem Medium enthaltenen Mikroorganismen aus Biofilmen aus Kläranlagen und/oder belebten Substanzen in Form von Belebtschlamm aus Kläranlagen oder Abwasser aus einem Hackschnitzel-Biofilter einer Kläranlage gewonnen wurden. Das Mikroorganismen enthaltende Medium kann insbesondere auch Biofilme aus Kläranlagen und/oder belebte Substanzen in Form von Belebtschlamm aus Kläranlagen oder Abwasser aus einem Hackschnitzel-Biofilter einer Kläranlage enthalten oder aus diesen bestehen.

Bei der Verwendung von Mikroorganismen aus Biofilmen und/oder belebten Substanzen in Form von Belebtschlamm aus Kläranlagen hat es sich als besonders praktikabel erwiesen, wenn der Belebtschlamm aus Kläranlagen ausgewählt ist aus der Gruppe bestehend aus Schlamm des aeroben oder anaeroben Behandlungsbeckens, Überschuss-Schlamm des aeroben oder anaeroben Behandlungsbeckens, Schlamm aus dem Faulturm, Primärschlamm und Kombinationen hiervon.

Neben der Verwendung von Belebtschlämmen aus kommunalen Kläranlagen sind auch Belebtschlämme aus industriellen Kläranlagen verwendbar, die üblicherweise frei von pathogenen Keimen und daher hygienisch unbedenklich sind.

Grundsätzlich funktioniert das erfindungsgemäße Verfahren mit einer Vielzahl von unterschiedlichen Mikroorganismen und insbesondere auch mit solchen, die nicht bereits an die Verstoffwechselung von cellulosehaltigen Materialien, wie beispielsweise Holz, gewöhnt sind. Dies gilt vor allem für den Fall, dass die Mikroorganismen aus einer Mikrobiozönose, wie beispielsweise einem Biofilm, stammen. Wie vorstehend erläutert, zeichnen sich Mikrobiozönosen durch eine sehr hohe Anpassungsfähigkeit an neue Nährstoffbedingungen aus.

In der Praxis hat sich gezeigt, dass bei der Verwendung von Mikroorganismen, die nicht bereits an die Verstoffwechselung von cellulosehaltigen Materialien, wie beispielsweise Holz, gewöhnt sind, eine "Eingewöhnungsphase" (lag-phase) des Systems zu beobachten ist. In dieser Eingewöhnungsphase findet zunächst nur ein langsamer Abbau von Holzextraktstoff, Vorläufersubstanzen von VOC und/oder VOC statt. In einer Art "Lernprozeß" erfolgt im Anschluss an die "Eingewöhnungsphase" durch Adaption und Selektion eine deutliche Steigerung des Abbaus von Holzextraktstoff, Vorläufersubstanzen von VOC und/oder VOC (log-phase).

Das Auftreten der beschriebenen Eingewöhnungsphase (lag-phase) kann vermieden oder reduziert werden, wenn das Mikroorganismen enthaltende Medium vor dessen Einsatz im erfindungsgemäßen Verfahren in einem Bioreaktor an Material auf Basis von Cellulose und/oder die Verstoffwechselung von Holzextraktstoffen, Vorläufersubstanzen von VOC und/oder VOC adaptiert wurde. Hierbei kann das Material auf Basis von Cellulose insbesondere identisch sein mit dem zu behandelnden und vorstehend beschriebenen Holz enthaltenden Vor- oder Zwischenprodukt des Holzwerkstoffs.

Eine solche Prä-Adaption an die Verstoffwechselung von Holzextraktstoffen, Vorläufersubstanzen von VOC und/oder VOC führt zu einer Beschleunigung des Verfahrens und zu einer weiteren Verbesserung der VOC-Reduktion über die gesamte Bandbreite an VOC-Spezies. Dies ist wahrscheinlich darauf zurückzuführen, dass die prä-adaptierten Mikroorganismen schon ihren Stoffwechsel entsprechend umstellen konnten, bzw. solche Mikroorganismen sich bevorzugt vermehren konnten, die auch oder besonders gut Holzextraktstoff, Vorläufersubstanzen von VOC und/oder VOC verstoffwechseln können.

Die Prä-adaption kann beispielsweise dadurch erfolgen, dass die in dem erfindungsgemäßen Medium enthaltenen Mikroorganismen zunächst kultiviert werden und dabei gängige Abwassertypen der Holzwerkstoffindustrie, insbesondere Abwasser aus der Aufbereitung von Holz durch Dämpfen, Kochen, Zerfasern oder Trocknen, stammt (beispielsweise Stopfschneckenaustragswasser, Wasser aus der Austragsschnecke des MDF Kochers, Wasser aus dem Regenrückhaltebecken, Abwasser der Stammwäsche etc.) dem Kultivierungsansatz beigemengt werden. Ziel ist es dabei, die Mikroorganismen bereits an holzrelevante Inhaltsstoffe zu gewöhnen. Dies führt zum einen zur Selektion (bevorzugte Anreicherung) von geeigneten Mikroorganismen und zum anderen zur Ausbildung und Induzierung geeigneter Enzymsysteme in den Mikroorganismen, welche Holzextraktstoff, Vorläufersubstanzen von VOC und/oder VOC metabolisieren können.

Gemäß einer besonderen Ausführungsform der Erfindung werden die Mikroorganismen nach und/oder während der Durchführung des erfindungsgemäßen Verfahrens wiedergewonnen und weiterkultiviert. Die so gewonnenen Mikroorganismen können in einer weiteren Anwendung des erfindungsgemäßen Verfahrens wiederverwendet werden. Hierdurch kann sich über mehrere Anwendungen des erfindunsgemäßen Verfahrens hinweg eine speziell adaptierte Mikrobiozönose bilden, die optimal auf die Verstoffwechselung von Holzextraktstoff, Vorläufersubstanzen von VOC und/oder VOC eingestellt ist.

Prinzipiell sind dem Fachmann verschiedene Quellen für Mikroorganismen und insbesondere Mikrobiozönosen und Biofilme, die geeignet sind, in dem erfindungsgemäßen Verfahren eingesetzt zu werden, bekannt. Für viele Anwendungen ist es besonders vorteilhaft, wenn die in dem Medium enthaltenen Mikroorganismen mengenmäßig (bezogen auf die Gesamtzahl der Mikroorganismen Einzellebewesen) zu mindestens etwa 50 %, bevorzugt mindestens etwa 80 % und noch bevorzugter mindestens etwa 90 % Bakterien sind. Dies ist in den meisten natürlichen Mikroorganismenpopulationen, wie sie beispielsweise in Abwasser und/oder belebten Filterrückständen aus der Aufbereitung von Abwasser vorkommen, der Fall.

Hefen haben sich für viele Anwendungen als eher ungeeignet erwiesen. Demnach sieht eine Ausführungsform der Erfindung vor, dass die in dem erfindungsgemäßen Medium enthaltenen Mikroorganismen mengenmäßig (bezogen auf die Gesamtzahl der Mikroorganismen Einzellebewesen) höchstens zu etwa 20 %, bevorzugt zu höchstens etwa 5 % und besonders bevorzugt höchstens zu etwa 1 % aus Hefen bestehen.

Es hat sich als besonders vorteilhaft erwiesen, wenn das Mikroorganismen enthaltende Medium mindestens einen Bakterienstamm enthält, der ausgewählt ist aus der Gruppe bestehend aus Enterobacteriacea, insbesondere Enterobacter chloacae, Klebsiella sp., Serratia ficaria, Pantoea spp., Pseudomonadacea, wie insbesondere Pseudomonas sp. und Bacillus sp.

Praktische Versuche haben gezeigt, dass sich optimale Ergebnisse einstellen, wenn das erfindungsgemäße Medium die Mikroorganismen in einer Zahl von mindestens $10^4$ Mikroorganismen/ml, bevorzugt mindestens $10^5$ Mikroorganismen/ml und noch bevorzugter mindestens $10^6$ Mikroorganismen/ml enthält.

Dabei kann die Mikroorganismenzahl/ml durch Auszählung der in einer Probe vorhandenen Mikroorganismen (Einzellebewesen) in einer Thoma Zählkammer ermittelt werden. Eine solche Zählkammer ist dem Fachmann bekannt und dient der lichtmikroskopischen Zählung aller Arten von kleinen Teilchen, die sich in einer Teilchensuspension befinden. Zählkammern werden besonders für die Quantifizierung von Zellen und Mikroorganismen in der Medizin und Biologie angewendet. Liegen die Mikroorganismen in dem Medium flokkuliert, also als Aneinanderballung kleinerer Gruppen von Mikroorganismen, oder in Form eines intakten oder zerkleinerten Biofilms vor, sollte die Probe vor der Auszählung kurz gevortext oder sonifiziert werden, um eine Trennung in Einzellebewesen zu erhalten.

Je nach Typ der auszuzählenden Mikroorganismen wird dann eine gewisse Anzahl Groß- oder Gruppenquadrate der Thomakammer ausgezählt und daraus ein Mittelwert gebildet. Multipliziert man diesen Wert mit einem entsprechenden Faktor (Kehrwert des Produkts aus Quadratfläche und Kammerhöhe), erhält man die Mikroorganismenzahl pro Volumeneinheit.

Dabei wird folgende Auswertungsformel angewendet:

$$\text{Teilchen pro µl Volumen} = \text{ausgezählte Teilchen}/(\text{ausgezählte Fläche }(mm^2) \cdot \text{Kammertiefe }(mm) \cdot \text{Verdünnung}).$$

Wird die Gesamtkeimzahl nach TrinkwV als Maßstab herangezogen (siehe oben), so enthält das erfindungsgemäße Medium die Mikroorganismen bevorzugt in einer Gesamtkeimzahl von mindestens etwa $10^3$ KbE/ml oder mindestens etwa $10^4$ KbE/ml, bevorzugt mindestens etwa $10^5$ KbE/ml und noch bevorzugter mindestens etwa $10^6$ KbE/ml enthält. Bei derart hohen Keimzahlen muss bei dem in der TrinkwV beschrieben mikrobiologischen Untersuchungsverfahren eine entsprechend hohe Verdünnung eingesetzt werden, damit einzelne Kolonien auf dem Agar-Nährboden unterschieden werden können. Über den gewählten Verdünnungsfaktor kann anschließend wieder auf die Gesamtkeimzahl in KbE/ml hochgerechnet werden.

In Einsatzversuchen hat es sich ferner als besonders vorteilhaft erwiesen, wenn bei der Behandlung mit dem Mikroorganismen enthaltenden Medium eine Mikroorganismendichte von mindestens etwa $1 \times 10^3$, vorzugsweise mindestens etwa $1 \times 10^4$, besonders bevorzugt mindestens etwa $1 \times 10^5$ oder mindestens etwa $1 \times 10^6$ Mikroorganismen Einzellebewesen pro g des Holz enthaltenden Vor- oder Zwischenprodukts vorliegt. Als besonders praxisgerecht hat sich bei der Behandlung mit dem Mikroorganismen enthaltenden Medium eine Mikroorganismendichte von etwa $1 \times 10^3$ bis etwa $1 \times 10^{12}$, insbesondere von etwa $1 \times 10^5$ bis etwa $1 \times 10^{10}$ Mikroorganismen Einzellebewesen pro g des Holz enthaltenden Vor- oder Zwischenprodukts herausgestellt. Die Mikroorganismendichte kann berechnet werden, indem zunächst die Mikroorganismenzahl/ml des eingesetzten Mikroorganismen enthaltenden Mediums wie vorstehend beschrieben ausgezählt wird. Anschließend kann über die während der Behandlung herrschende Holzfeuchte die Anzahl der Mikroorganismen Einzellebewesen pro g des Holz enthaltenden Vor- oder Zwischenprodukts berechnet werden. Genauso wie die oben beschriebene Holzfeuchte ist auch die Mikroorganismendichte bezogen auf das Holztrockengewicht (Darrgewicht).

Gemäß einer weiteren Ausführungsform der Erfindung können dem Mikroorganismen enthaltenden Medium ausgewählte stoffwechselstimulierende Additive zugesetzt werden. Solche Additive sind dem Fachmann aus der Verwendung von Mikrobiozönosen und Biofilmen bei der biologischen Abwasseraufbereitung bekannt. Beispiele für geeignete Additive, die der Stimulierung der mikrobiellen Biomasse dienen, sind Vitamine, Glucose und Stickstoff-haltige Nährstoffe, die eine ausreichende Stickstoff-Versorgung der Mikroorganismen sicherstellen. Es hat sich gezeigt, dass der Zusatz derartiger Additive die Abbauleistung in Bezug auf VOC und deren Vorläufersubstanzen entscheidend beeinflussen kann.

Ferner können zusätzlich weitere, ggf. gereinigte, Mikroorganismen und deren Exoenzyme (z. B. ligninabbauende Pilze und deren Enzymspektren) gezielt in das Mikroorganismen enthaltende Medium eingetragen werden.

Das erfindungsgemäße Verfahren zur Herstellung eines Holzwerkstoffs dient insbesondere dazu, einen Holzwerkstoff mit einem verringerten Gehalt an Holzextraktstoff, Vorläufersubstanzen von VOC und/oder VOC herzustellen. Die mit dem erfindungsgemäßen Verfahren erzielbare Reduktion des Gehalts an Holzextraktstoff, Vorläufersubstanzen von VOC und/oder VOC erfolgt vermutlich durch biochemische Prozesse des Primär- und Sekundärstoffwechsels der in dem erfindungsgemäßen Medium enthaltenen Mikroorganismen.

In der Praxis können mit dem erfindungsgemäßen Verfahren optimale Ergebnisse erzielt werden, wenn der Holzwerkstoff ein gepresster Holzwerkstoff, insbesondere eine Faser- oder OSB-Platte, ist.

Wenn hier von "gepresstem Holzwerkstoff" die Rede ist, so sind damit alle Holzwerkstoffe gemeint, deren Herstellung mindestens einen Schritt umfasst, bei dem Holzpartikel unter Anwendung von Druck verdichtet werden. Dabei ist mit "Holzwerkstoff" jedes Material und jeder Gegenstand gemeint, der Holz oder holzartige Materialien enthält oder überwiegend aus diesen besteht. Die Bezeichnung "holzartige Materialien", wie sie hier verwendet wird, schließt alle lignocellulosehaltigen Materialien ein. Beispiele für holzartige Materialien im Sinne dieser Erfindung sind Getreidestroh, Hanf und/oder Flachs.

Gemäß einer besonderen Ausführungsform der Erfindung werden Holzpartikel als Holz enthaltendes Vor- oder Zwischenprodukt verwendet und das oben beschriebene Verfahren zur Herstellung des Holzwerkstoffs ist derart ausgestaltet, dass es mindestens die folgenden Schritte umfasst:

a) In Berührung bringen von Holzpartikeln mit dem Mikroorganismen enthaltenden Medium;
b) Inkubation des Materials aus Schritt a);
c) Trocknung der vorbehandelten Holzpartikel bei 80 bis 400 °C;
d) Beleimen der vorbehandelten Holzpartikel mit einem Bindemittel; und
e) Verpressen der Mischung zu einem gepressten Holzwerkstoff.

Dabei ist mit "Holzpartikel" jeder Gegenstand gemeint, der überwiegend aus Holz ist oder holzartiges Material enthält und der zur Herstellung eines Holzwerkstoffs durch Pressung geeignet ist. Unter Holzpartikeln im Sinne der Erfindung wird insbesondere jede Form von Fragmenten verstanden, die durch Zerkleinern von Cellulose enthaltenden Materialien erhältlich sind. Holzpartikel im Sinne dieser Erfindung sind insbesondere jede Form von kleinstückigem Holzmaterial, d.h. insbesondere Späne, OSB-Strands, Fasern und/oder Mehl. Gemäß einer bevorzugten Ausführungsform bestehen die Holzpartikel, insbesondere wenn es sich dabei um Späne, Fasern oder Strands handelt, überwiegend oder vollständig aus nativem Holz.

Die Schritte a) und b) stellen dabei die vorstehend beschriebene erfindungsgemäße Behandlung eines Holz enthaltenden Vor- oder Zwischenprodukts mit einem Mikroorganismen enthaltenden Medium dar. Insofern gilt das vorstehend zu den Ausgestaltungen dieser Behandlung, insbesondere zu möglichen Formen des in Berührung Bringens und der Inkubation, Gesagte entsprechend.

In Schritt c) findet die Trocknung der vorbehandelten Holzpartikel bei einer Temperatur von etwa 80°C bis etwa 400°C, bevorzugt von etwa 150°C bis etwa 350°C und besonders bevorzugt von etwa 150°C bis etwa 250°C statt. Eine Trocknung in letzterem Temperaturbereich hat sich als besonders wirksam erwiesen. Wenn hier von Trocknen oder Trocknung die Rede ist, so ist damit ein Erhitzen der behandelten Holzpartikel bei einer bestimmten Temperatur (Umgebungstemperatur) gemeint.

Dem Fachmann ist bekannt bzw. er kann durch einfaches Ausprobieren herausfinden, welche Restfeuchte der behandelten Holzpartikel nach der Trocknung für eine bestimmte Holzpartikel - Bindemittelkombination am besten geeignet ist. Als besonders praktikabel hat sich erwiesen, wenn in Schritt c) des erfindungsgemäßen Verfahrens das Erhitzen der Holzpartikel so lange erfolgt, bis die Holzpartikel eine Restfeuchte von 0,1 bis 10 %, insbesondere von 0,5 bis 6 %, bevorzugt von 1 bis 3 % erreicht haben.

Die Menge des in Schritt d) des erfindungsgemäßen Verfahrens eingesetzten Bindemittels beträgt vorzugsweise 0,01 bis 15 Gew.%, insbesondere 0,1 bis 10 Gew.%, noch bevorzugter 0,5 bis 7,5 Gew.%, bezogen auf das Holztrockengewicht (atro). Für viele Anwendungen ist es besonders praxisgerecht, wenn das Bindemittel in Schritt d) in einer Menge von 1 bis 15 Gew.-% bezogen auf das Holztrockengewicht (atro) eingesetzt wird.

Grundsätzlich ist das erfindungsgemäße Verfahren für eine Vielzahl von Bindemittel-Holzpartikel Kombinationen geeignet. Beispiele für im erfindungsgemäßen Verfahren einsetzbare Bindemittel sind Aminoplaste, Phenoplaste, Vinylacetate, Isocyanate, Epoxidharze und/oder Acrylharze. Besonders bevorzugte Bindemittel sind Harnstoff-Formaldehyd-Harz (UF), Melamin-Formaldehyd-Harz, Phenol-Formaldehyd-Harz (PF), Polyvinylacetat und/oder Weissleim. Gemäß einer bevorzugten Ausführungsform der Erfindung wird als Bindemittel in Schritt d) ein System auf Basis von Harnstoff-Formaldehyd-Harzen (UF), Melamin verstärkten Harnstoff-Formaldehyd-Harzen (MUF), Melamin-Harnstoff-Phenol- Formaldehyd-Harzen(MUPF), Phenol-Formaldehyd-Harzen (PF), polymere Diisocyanaten (PMDI) und/oder Isocyanaten eingesetzt.

Das in dem erfindungsgemäßen Verfahren eingesetzte Bindemittel kann ferner einen Härter enthalten. Alternativ oder zusätzlich kann das Härten auch durch Pressen des Verbundmaterials unter Wärmeeinwirkung erfolgen.

Grundsätzlich sind dem Fachmann verschiedene Methoden bekannt, beleimte Holzpartikel zu einem Verbundwerkstoff zu verpressen. Praktische Versuche haben gezeigt, dass bei dem erfindungsgemäßen Verfahren optimale Ergebnisse erzielt werden, wenn das Verpressen in Schritt e) bei einer Presstemperatur von mindestens etwa 150°C durchgeführt wird.

Die Auswahl an Holzpartikel-Arten, die in dem erfindungsgemäßen Verfahren eingesetzt werden können, ist nahezu unbegrenzt. Als besonders geeignet haben sich beispielsweise Späne, Fasern oder Strands aus nativem Holz erwiesen.

Gegenstand der Erfindung ist ferner auch ein Holzwerkstoff, der nach dem hier beschriebenen erfindungsgemäßen Verfahren und seinen verschiedenen Ausführungsformen hergestellt bzw. erhältlich ist.

Die Erfindung beinhaltet auch die Zwischenprodukte des erfindungsgemäßen Verfahrens. Diese umfassen insbesondere die behandelten Vor- oder Zwischenprodukte des Holzwerkstoffs.

Gegenstand der Erfindung ist ferner auch ein Mikroorganismen enthaltendes Medium zur Verringerung des Gehalts an Holzextraktstoff, Vorläufersubstanzen von VOC und/oder VOC in einem Material auf Basis von Cellulose, insbesondere Holz und/oder einem Holzwerkstoff, dadurch gekennzeichnet, dass das Mikroorganismen enthaltende Medium in einem Bioreaktor an Material auf Basis von Cellulose und/oder die Verstoffwechselung von Holzextraktstoffen, Vorläufersubstanzen von VOC und/oder VOC adaptiert worden ist.

Dabei können gemäß weiteren vorteilhaften Ausgestaltungen der Erfindung das Mikroorganismen enthaltende Medium, das Material auf Basis von Cellulose, der Holzwerkstoff, der Holzextraktstoff, die Vorläufersubstanzen von VOC, die VOC und die Adaption der Mikroorganismen wie vorstehend für das erfindungsgemäße Verfahren beschrieben sein.

Gegenstand der Erfindung ist schließlich auch die Verwendung eines Mikroorganismen enthaltenden Mediums zur Verringerung des Gehalts an Holzextraktstoff, Vorläufersubstanzen von VOC und/oder VOC in Holz oder einem Holzwerkstoff.

Nachfolgend wird die Erfindung anhand von Ausführungsbeispielen unter Bezugnahme auf die beigefügten Figuren näher beschrieben. Die Beispiele dienen der Illustration der Erfindung und schränken den Schutzumfang der Erfindung nicht ein.

In den Figuren zeigen:

Figur 1     eine GC-MS/SPME Analyse der Hexanal Fraktion von unbehandelten (control) und mit Kläranlagenproben (PS, Primärschlamm; ES, Eindickungsschlamm) behandelten Holzpänen.

Figur 2     eine GC-MS/SPME Analyse der Pentanal Fraktion von unbehandelten (control) und mit Kläranlagenproben (PS, Primärschlamm; ES, Eindickungsschlamm) behandelten Holzpänen.

Figur 3     eine GC-MS/SPME Analyse der Hexanal Fraktion von unbehandelten (control) und mit Holzindustrie Abwasser (WSR, Stopfschneckenaustragswasser eines Sägespan-Refiners; WCR, Stopfschnecken-austragswasser eines Hackschnitzel-Refiners) behandelten Holzpänen.

Figur 4     eine GC-MS/SPME Analyse der α-Pinen Fraktion von unbehandelten (control) und mit einer Kläranlagen-Hackschnitzel-Biofilter Probe (BF) behandelten Holzpänen.

Figur 5     eine GC-MS/SPME Analyse der β-Pinen Fraktion von unbehandelten (control) und mit einer Kläranlagen-Hackschnitzel-Biofilter Probe (BF) behandelten Holzpänen.

Figur 6     eine GC-MS/SPME Analyse der δ-3-Caren Fraktion von unbehandelten (control) und mit einer Kläranlagen-Hackschnitzel-Biofilter Probe (BF) behandelten Holzpänen.

Figur 7    eine GC-MS/SPME Analyse der Hexanal Fraktion von unbehandelten (control) und mit Holzindustrie Abwasser (WSR, Stopfschneckenaustragswasser eines Sägespan-Refiners; WCR, Stopfschnecken-austragswasser eines Hackschnitzel-Refiners) behandelten Holzstrands.

Figur 8    eine MS Analyse der Aldehyd Emission von Laborplatten, die aus unbehandelten (control) und mit Holzindustrie Abwasser (WSR, Stopfschnecken-austragswasser eines Sägespan-Refiners; WCR, Stopfschneckenaustragswasser eines Hackschnitzel-Refiners) behandelten Holzstrands hergestellt wurden.

Das Prinzip der Erfindung soll im Folgenden an Beispielen näher erläutert werden.

Beispiel 1

In einem Laborversuch wurden Kiefern-Holzspäne mit verschiedenen Mikroorganismen enthaltenden Medien behandelt und anschließend der Gehalt einzelner VOC (Hexanal und Pentanal) bestimmt.

Als Mikroorganismen enthaltendes Medium wurden verschiedene Abwasserproben verwendet. Alle Abwasserproben dienten in dem Versuch sowohl als Nahrungsquelle als auch als mikrobielles Inokulum.

Ein Teil der Abwasserproben stammt aus einer kommunalen Kläranlage in Wien, Österreich. Die Proben wurden von mehreren Standorten des Klärungkreislaufes wie Erstbehandlung (Primärschlamm, PS) und Eindickung (Überschussschlamm, ES) entnommen.

Außerdem wurden Holzindustrie-Abwässer getestet, die aus einer Holzwerkstoffproduktion stammen. Hierfür wurde Stopfschneckenaustragswasser eines Sägespan-Refiners (WSR) und Stopfschneckenaustragswasser eines Hackschnitzel-Refiners (WCR) während des routinemäßigen Produktionsprozesses gesammelt.

In der vorwiegend bakteriellen Mikrobiozönose der Kläranlagenabwässer (PS und ES) waren unter anderem Arten der Familie der *Enterobacteriacea* wie *Enterobacter chloacae, Klebsiella sp, Serratia ficaria, Pantoea spp.* sowie der *Pseudomonadacea* wie *Pseudomonas sp* nachweisbar.

In den Mikrobiozönosen der Holzindustrie Abwässer (WSR und WCR) waren unter anderem die Bakterienspezies *Klebsiella sp, Pseudomonas sp.* und das ubiquitäre Bakterium *Bacillus sp.* nachweisbar.

Jeweils 1 Gramm (Feuchtgewicht) Kiefern-Holzspäne wurde in 20 ml Headspace-Fine-Pitch-Thread-Fläschchen (VWR®) abgewogen und mit einem membranhaltigen Schraubverschluss verschlossen. Alle Fläschchen wurden nach standardisiertem Sterilisationsprotokoll für 15 Minuten bei 121 ° C autoklaviert.

Nach dem Abkühlen auf Raumtemperatur wurden die Röhrchen mit 1 ml des jeweiligen Abwassers inokuliert (WCR, WSR, PS, und ES). Die Mikroorganismendichte betrug etwa 4 x $10^9$ Mikroorganismen Einzellebewesen pro g Holztrockengewicht der Kiefern-Holzspäne. Zur Untersuchung des Einflusses der Abwassernährstoffbestandteile selbst wurden zusätzliche Röhrchen mit sterilisiertem Abwasser vorbereitet (WCR sterile, WSR sterile, PS sterile, und ES sterile). Als weitere Kontrolle wurde ein Röhrchen mit 1 ml sterilem Leitungswasser versetzt (control). Alle Versuche wurden als Triplikat durchgeführt.

Die Röhrchen wurden für 7 Tage bei 24°C inkubiert und anschließend die VOC-Emissionen der Kiefern-Holzspäne qualitativ und halbquantitativ mittels SPME / GC-MS-Analyse analysiert.

SPME/GC-MS-Analytik:

Für die SPME/GC-MS-Analyse wurde eine 75 $\mu$m CAR / PDMS-Faser (Supelco) verwendet. Die Faser wurde bei 30°C für 25 min in die Headspace-Flaschen eingeführt. Die Desorption lief in einem Agilent 7890A bei 40°C für 400 s. Auf die MS HP5-Säule (30 m x 0,25 mm ID x 0,25 $\mu$m) wurden 1 $\mu$l Probenvolumen bei 40°C aufgebracht. Die Ofentemperatur wurde für 4 min bei 40°C gehalten, stieg dann linear von auf 110°C bei 4°C min$^{-1}$ und stieg linear weiter auf 310°C bei 30°C min$^{-1}$, wo sie für 2 min gehalten wurde. Das Massenspektrometer war auf einen Massenbereich von 37 bis 700 m / z eingestellt. Mit Hilfe von im Handel erhältlichen Datenbanken (Wiley, NIST) konnten die Substanzen in etwa bestimmt werden. Anschließend wurden die Peakflächen zwischen den einzelnen Proben (beimpft / unbeimpft / steril und unterschiedliche Abwässer) verglichen.

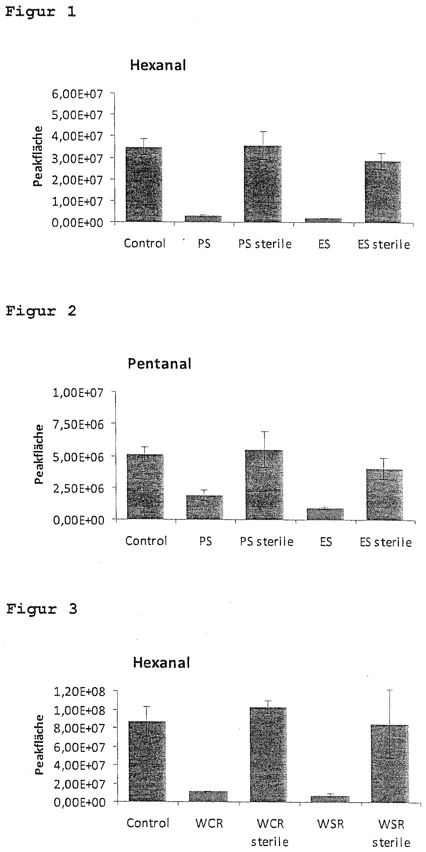

Der Vergleich der Hexanal Fraktion in den mit Kläranlagenabwasser (PS und ES) behandelten Proben ist als Balkendiagramm in Figur 1 abgebildet. Ein entsprechender Vergleich der Pentanal Fraktion ist in Figur 2 abgebildet.

Der Vergleich der Hexanal Fraktion in den mit Holzindustrie Abwasser (WSR und WCR) behandelten Proben ist als Balkendiagramm in Figur 3 abgebildet.

Im Ergebnis konnte eine signifikante Reduktion der AldehydEmissionen bei den mit Holzindustrieabwasser und Klärabwasser behandelten Proben beobachtet werden. Insbesondere zeigte sich eine signifikante Verringerung ihrer Pentanal und Hexanal-Emissionen im Vergleich zu nichtbehandelten Proben. Die Ergebnisse deuten darauf hin, dass das mikrobielle Wachstum zum Verbrauch von VOC (insbesondere Aldehyden) und deren Vorläufersubstanzen (insbesondere Fettsäuren) führt.

SPME-Analytik:

Die behandelten Späne und die Späne aus den unbehandelten Kontrollproben wurden gefriergetrocknet und danach für die Extraktionsanalyse mittels Solid Phase Micro Extraction (SPME) verwendet.

Die Extraktion wurde mit einem Büchi SpeedExtraktor E-916 durchgeführt. Das Verfahren bestand aus vier Extraktionszyklen (jeweils 5 Minuten) und einem Extraktionsgemisch von 95/5 Aceton-Wasser (v/v) bei 110 bar und 110 °C. Aus den dadurch enthaltenen Extrakten wurde jeweils 1,5 ml Probevolumen in ein Pyrex Röhrchen überführt und mit 100 $\mu$l internen Standard (ISTD) versetzt. Der interne Standard bestand aus 6 mg Heneicosansäure, 9 mg Cholesterylpalmitat, 9 mg 1,3-Dipalmitoyl-2-deyl-glycerd, 9 mg Betulin gelöst in 12 ml Aceton.

Nach Vakuum-Verdampfung des Lösungsmittels folgte die Derivatisierung mit 80 $\mu$l BSTFA (N, O-Bis-trimethylsilyltrifluoracetamid, Sigma), 20 $\mu$l TMCS (Trimethylchlorsilan, Sigma) und 20 $\mu$l Pyridin bei 70 °C für 45 min. in den verschlossenen Pyrex Röhrchen.

Die Proben wurden bei 270 °C injiziert und auf einer MS HP5-Säule (30 m x 0,25 mm ID x 0,25 $\mu$m) getrennt. Die Ofentemperatur wurde für 1 min bei 100 °C gehalten, stieg dann linear von auf 325 °C bei 5 °C min$^{-1}$ an und wurde dann für 14 min gehalten. Das Massenspektrometer war auf einen Massenbereich von 37 bis 700 m / z eingestellt. Eine ungefähre Quantifizierung war über das Multiplizieren der bekannten Menge des ISTD mit dem Quotienten aus der Substanz Peakfläche und der ISTD Peakfläche möglich. Dies geschah unter der Annahme, dass die Response-Faktoren für alle analysierten Substanzen identisch waren und dass die Kalibrierungskurven im Nachweisbereich linear waren.

Beispiel 2

Kiefern-Holzspäne wurden wie in Beispiel 1 beschrieben behandelt und untersucht, nur dass als Mikroorganismen enthaltendes Medium eine Probe aus dem vorgelagerten Hackschnitzel-Biofilter (BF) einer kommunalen Kläranlage verwendet wurde und als VOCs verschiedene Terpene ($\alpha$-Pinen, $\beta$-Pinen und $\delta$-3-Caren) mittels GC-MS/SPME Analyse untersucht wurden.

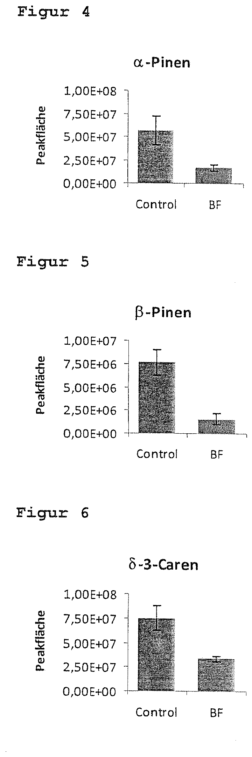

Der Vergleich der $\alpha$-Pinen, $\beta$-Pinen und $\delta$-3-Caren Fraktion in den mit der Hackschnitzel-Biofilter Probe (BF) behandelten Holzspäne ist als Balkendiagramm in Figuren 4, 5 und 6 abgebildet.

Im Ergebnis konnte eine signifikante Reduktion der Terpen-Emissionen bei den mit der Hackschnitzel-Biofilter Probe (BF) behandelten Holzspänen beobachtet werden. Insbesondere zeigte sich eine signifikante Verringerung ihrer $\alpha$-Pinen-, $\beta$-Pinen- und $\delta$-3-Caren-Emissionen im Vergleich zu nichtbehandelten Proben. Die Ergebnisse deuten darauf hin, dass das mikrobielle Wachstum auch zum Verbrauch von Terpen-VOCs und/oder deren Vorläufersubstanzen führt.

Beispiel 3

In einem weiteren Versuch wurde im größeren Maßstab gearbeitet und aus den behandelten Holzpartikel Laborplatten gepresst.

Zur Behandlung wurden die gleichen Holzindustrie Abwässer (WCR und WSR) verwendet wie in Beispiel 1.

36 kg frische Kiefernholzstrands mit einem Feuchtegehalt von 58% wurden äquivalent auf 36 Polyethylen Zip-Lock Beutel (40 x 60 cm$^2$, 50 um Dicke) aufgeteilt und anschließend gammasterilisiert (Dosis: 25,5 kGy min nach ISO 9001:2008). Je 12 der sterilisierten Beutel wurden mit dem Abwasser WSR und WCR inokuliert. Zu jedem Aliquot wurden 200 ml des jeweiligen Abwassers zugegeben. Die Mikroorganismendichte betrug etwa 1 x 10$^9$ Mikroorganismen Einzellebewesen pro g Holztrockengewicht der Kiefernholzstrands. Die Holzfeuchte betrug 243 %.

12 Kontrollproben wurden stattdessen mit 200 ml sterilisiertem Wasser versetzt. Alle Beutel wurden für 6 Wochen bei 23° C inkubiert.

Anschließend wurden die Aliquots jeder Gruppe (Kontrolle, WSR und WCR) vereinigt und nacheinander in einem rotierenden Trommeltrockner für ca. 4 Stunden bei Zuluft von ca. 35 m$^3$ h$^{-1}$ und einer Zulufttemperatur von 180 ° C getrocknet. Der Feuchtigkeitsgehalt des getrockneten Materials betrug ca. 4,0% (unbehandelte Strands = control) und 4,8/5,2% (WSR/WCR behandelte Strands).

Der VOC-Gehalt der mit Holzindustrieabwasser behandelten Proben und der Kontrollproben wurde wie oben beschrieben mittels GC-MS bestimmt.

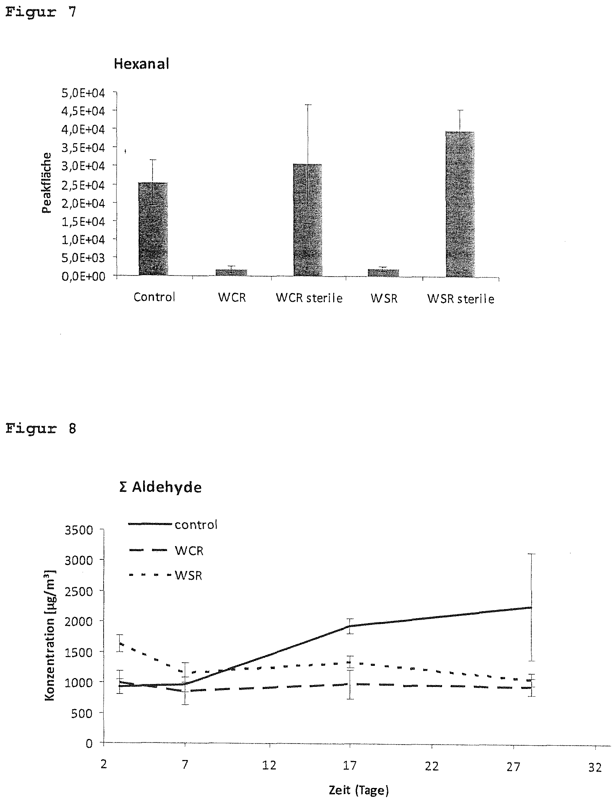

Ein Vergleich der Hexanal Fraktion der mit Holzindustrie Abwasser (WSR und WCR) behandelten Strands ist als Balkendiagramm in Figur 7 abgebildet. Im Ergebnis zeigte sich eine signifikante Verringerung der Hexanal-Werte im Vergleich zu nichtbehandelten Strands.

Aus den behandelten Strands und den Kontrollproben wurden anschließend Laborplatten nach dem in Tabelle 1 angegebenen Protokoll hergestellt.

Tabelle 1

| Pressparameter | | |
|---|---|---|
| Obergrenze Druck | [bar] | 150.0 |
| max. Druck | [bar] | 74.4 |
| Temperatur unten | [°C] | 191.0 |
| Temperatur oben | [°C] | 195.1 |
| Distanz | [mm] | 12.0 |
| Pressfaktor | [s/mm] | 10.8 |
| Ist Beleimfeuchte - Presse | [%] | 10.5 |
| **Chemie** | | |
| Leim-Bezeichnung | - | PMDI |
| Härter-Type | - | |

Aus den Laborplatten wurden 41 Tage nach Herstellung vier Probenscheiben (Ø 43,5 mm) je Laborplatte für die VOC Messungen geschnitten. Die Probenscheiben wurden in separate pCTE™ Mikrokammern/Thermal Extractors (Markes®) gelegt und der VOC Gehalt an Tag 3, 7, 17 und 28 nach Mikrokammereinlagerung gemessen. Die flächenspezifische Luftaustauschrate wurde auf $0,5 \text{ m}^3 \times \text{m}^{-2} \times \text{h}^{-1}$ eingestellt. Ansonsten wurden die Testparameter eingestellt und das VOC-Messprotokoll verwendet, wie in Stratev, D., Gradinger, C., Ters, T., Fackler, K., Kuncinger, T., Srebotnik, E. (2011) "Fungal pretreatment of pine wood to reduce the emission of volatile organic compounds", Holzforschung (65) beschrieben.

Einzelne VOCs wurden per Massenspektrometrie (MS) identifiziert. Dabei erfolgte die Peak Integration semiautomatisch durch die Agilent MSD Chemstation software (Agilent). Der Gehalt an Pentanal, Hexanal und Octanal wurde quantifiziert, addiert und als "Summe Aldehyde" dargestellt. Die Quantifizierung erfolgte mit externer Kalibrierung mit authentischen Standards und d8-Toluene als internem Standard.

Als beispielhaftes Ergebnis der Mikrokammermessungen ist in Abbildung 8 der Vergleich der "Summe Aldehyde" Fraktionen der aus mit Holzindustrie Abwasser (WSR und WCR) behandelten Strands hergestellten Platten abgebildet.

Platten aus behandelten Strands zeigten eine signifikante Verringerung ihrer Aldehyd-Emissionen im Vergleich zu Platten aus nichtbehandelten Strands. Die Ergebnisse deuten darauf hin, dass das mikrobielle Wachstum zum Verbrauch von VOC (insbesondere Aldehyden) und deren Vorläufersubstanzen (insbesondere Fettsäuren) führt.

**Patentansprüche**

**1.** Verfahren zur Herstellung eines Holzwerkstoffs, wobei der Holzwerkstoff eine Holzplatte oder Holzformteil ist und einen Verbundwerkstoff aus einzelnen Holzpartikeln oder einen Werkstoff aus Massivholz darstellt, **dadurch gekennzeichnet, dass** ein Holz enthaltendes Vor- oder Zwischenprodukt des Holzwerkstoffs mit einem, eine Pluralität von unterschiedlichen Mikroorganismenarten enthaltenden Medium behandelt wird, wobei

(i) die Behandlung den Schritt umfasst, dass die Pluralität von unterschiedlichen Mikroorganismenarten dem Holz enthaltenden Vor- oder Zwischenprodukt aktiv zugeführt bzw. mit diesem in Berührung gebracht wird; und
(ii) vom Begriff der "Behandlung" keine Prozesse umfasst sind, in denen Mikroorganismen, die schon von vorneherein auf oder in dem Holz enthaltenden Vor- oder Zwischenprodukte des Holzwerkstoffs vorhanden waren, auf diesen im Laufe der Zeit einwirken und ihn zu zersetzen beginnen; und
(iii) das Mikroorganismen enthaltende Medium Biofilme und/oder belebte Substanzen in Form von Belebtschlamm aus Kläranlagen oder Abwasser aus einem Hackschnitzel-Biofilter einer Kläranlage enthält oder aus diesen besteht und mindestens einen Bakterienstamm enthält, der ausgewählt ist aus der Gruppe bestehend aus *Enterobacteriacea, Klebsiella sp., Serratia ficaria, Pantoea spp., Pseudomonadacea* und *Bacillus sp..*

**2.** Verfahren nach Anspruch 1, **dadurch gekennzeichnet, dass** die in dem Medium enthaltenen Mikroorganismen eine Mikrobiozönose sind.

3. Verfahren nach Anspruch 1 oder 2, **dadurch gekennzeichnet, dass** die in dem Medium enthaltenen Mikroorganismen aus Abwasser und/oder Filterrückständen aus der Aufbereitung dieses Abwassers gewonnen wurden.

4. Verfahren nach einem der voranstehenden Ansprüche, **dadurch gekennzeichnet, dass** das Mikroorganismen enthaltende Medium Abwasser und/oder Filterrückstände aus der Aufbereitung dieses Abwassers enthält oder hieraus besteht.

5. Verfahren nach Anspruch 3 oder 4, **dadurch gekennzeichnet, dass** das Abwasser ausgewählt ist aus der Gruppe bestehend aus Niederschlagswasser, häusliches Abwasser, kommunales Abwasser, Industrieabwasser, Abwasser von Wasseraufbereitungsanlagen und Kombinationen hiervon.

6. Verfahren nach einem der Ansprüche 3 bis 5, **dadurch gekennzeichnet, dass** das Abwasser ein Abwasser ist, dass mit Holz und/oder Holzbestandteilen in Berührung gekommen ist, insbesondere Abwasser aus der Holz- und/oder holzverarbeitenden Industrie.

7. Verfahren nach Anspruch 6, **dadurch gekennzeichnet, dass** das Abwasser aus der Holzwerkstoffproduktion, insbesondere aus der Aufbereitung von Holz durch Dämpfen, Kochen, Zerfasern oder Trocknen, stammt.

8. Verfahren nach einem der voranstehenden Ansprüche, **dadurch gekennzeichnet, dass** die in dem Medium enthaltenen Mikroorganismen aus Biofilmen und/oder belebten Substanzen in Form von Belebtschlamm aus Kläranlagen oder Abwasser aus einem Hackschnitzel-Biofilter einer Kläranlage, gewonnen wurden.

9. Verfahren nach Anspruch 8, **dadurch gekennzeichnet, dass** der Belebtschlamm aus Kläranlagen ausgewählt ist aus der Gruppe bestehend aus Schlamm des aeroben oder anaeroben Behandlungsbeckens, Überschuss-Schlamm des aeroben oder anaeroben Behandlungsbeckens, Schlamm aus dem Faulturm, Primärschlamm und Kombinationen hiervon.

10. Verfahren nach einem der voranstehenden Ansprüche, **dadurch gekennzeichnet, dass** das Mikroorganismen enthaltende Medium vor dessen Einsatz im erfindungsgemäßen Verfahren in einem Bioreaktor an Material auf Basis von Cellulose und/oder die Verstoffwechselung von Holzextraktstoffen, Vorläufersubstanzen von VOC und/oder VOC adaptiert wurde.

11. Verfahren nach einem der voranstehenden Ansprüche, **dadurch gekennzeichnet, dass** die in dem Medium enthaltenen Mikroorganismen zu mindestens etwa 50 %, bevorzugt mindestens etwa 80 % und noch bevorzugter mindestens etwa 90 % Bakterien sind.

12. Verfahren nach einem der voranstehenden Ansprüche, **dadurch gekennzeichnet, dass** das Mikroorganismen enthaltende Medium mindestens einen Bakterienstamm enthält, der ausgewählt ist aus der Gruppe bestehend aus *Enterobacter chloacae* und *Pseudomonas sp.*.

13. Verfahren nach einem der voranstehenden Ansprüche, **dadurch gekennzeichnet, dass** das Medium Mikroorganismen in einer Gesamtzahl von mindestens etwa $10^4$ Mikroorganismen/ml, bevorzugt mindestens etwa $10^5$ Mikroorganismen/ml und noch bevorzugter mindestens etwa $10^6$ Mikroorganismen/ml enthält.

14. Verfahren nach einem der voranstehenden Ansprüche, **dadurch gekennzeichnet, dass** das Holz enthaltende Vor- oder Zwischenprodukt des Holzwerkstoffs bei der Behandlung mit dem Mikroorganismen enthaltenden Medium eine Holzfeuchte von höchstens 300 %, bevorzugt höchsten 250 % und besonders bevorzugt von 130 bis 250 %, aufweist.

15. Verfahren nach einem der voranstehenden Ansprüche, **dadurch gekennzeichnet, dass** bei der Behandlung mit dem Mikroorganismen enthaltenden Medium eine Mikroorganismendichte von mindestens etwa $1 \times 10^3$, vorzugsweise mindestens etwa $1 \times 10^4$, besonders bevorzugt mindestens etwa $1 \times 10^5$ oder mindestens etwa $1 \times 10^6$ Mikroorganismen Einzellebewesen pro g des Holz enthaltenden Vor- oder Zwischenprodukts vorliegt.

16. Verfahren nach einem der voranstehenden Ansprüche, **dadurch gekennzeichnet, dass** das Verfahren zu einer Verringerung des Gehalts an Holzextraktstoff, Vorläufersubstanzen von VOC und/oder VOC im Holzwerkstoff führt.

17. Verfahren nach einem der voranstehenden Ansprüche, **dadurch gekennzeichnet, dass** der Holzwerkstoff ein gepresster Holzwerkstoff, insbesondere eine Faser- oder OSB-Platte, ist.

18. Verfahren nach Anspruch 17, **dadurch gekennzeichnet, dass** das Holz enthaltende Vor- oder Zwischenprodukt des Holzwerkstoffs Holzpartikel sind und das Verfahren mindestens folgende Schritte umfasst:

   **a)** In Berührung bringen von Holzpartikeln mit dem Mikroorganismen enthaltende Medium;
   **b)** Inkubation des Materials aus Schritt a);
   **c)** Trocknung der vorbehandelten Holzpartikel bei 80 bis 400 °C;
   **d)** Beleimen der vorbehandelten Holzpartikel mit einem Bindemittel; und
   **e)** Verpressen der Mischung zu einem gepressten Holzwerkstoff.

19. Verwendung eines eine Pluralität von unterschiedlichen Mikroorganismenarten enthaltenden Mediums in einem Verfahren gemäß Ansprüche 1-18 zur Verringerung des Gehalts an Holzextraktstoff, Vorläufersubstanzen von VOC und/oder VOC in Holz oder einem Holzwerkstoff.

## Claims

1. Method for the production of a wooden material, wherein the wooden material is a wooden board or a wooden moulded part and represents a composite material made from individual wooden particles or a material made from solid wood, **characterised in that** a precursor or intermediate product of the wooden material containing wood is treated with a medium containing a plurality of different microorganism types, wherein

   (i) the treatment comprises the step that the plurality of different microorganism types is actively supplied to the precursor or intermediate product containing wood or is brought into contact with this; and
   (ii) by the term "treatment", no processes are comprised in which microorganisms which were already present from the outset on or in the precursor or intermediate products of the wooden material containing wood act on this over the course of time and begin to destroy it; and
   (iii) the medium containing microorganisms contains biofilms and/or activated substances in the form of activated sludge from sewage treatment plants or waste water from a wood chip biofilter of a sewage treatment plant or consists of these and contains at least one bacterial strain which is selected from the group consisting of *Enterobacteriaceae, Klebsiella sp., Serratia ficaria, Pantoea spp., Pseudomonadaceae and Bacillus sp.*

2. Method according to Claim 1, **characterised in that** the microorganisms contained in the medium are a microbiocoenosis.

3. Method according to Claim 1 or 2, **characterised in that** the microorganisms contained in the medium were obtained from waste water and/or filter residues from the processing of this waste water.

4. Method according to any one of the preceding claims, **characterised in that** the medium containing microorganisms contains waste water and/or filter residues from the treatment of this waste water or consists thereof.

5. Method according to Claim 3 or 4, **characterised in that** the waste water is selected from the group consisting of rainwater, domestic waste water, communal waste water, industrial waste water, waste water from water treatment plants and combinations thereof.

6. Method according to any one of Claims 3 to 5, **characterised in that** the waste water is a waste water that is brought into contact with wood and/or wooden components, in particular waste water from the wood and/or wood processing industry.

7. Method according to Claim 6, **characterised in that** the waste water originates from wooden material production, in particular from the processing of wood by steaming, boiling, defibring or drying.

8. Method according to any one of the preceding claims, **characterised in that** the microorganisms contained in the medium were obtained from biofilms and/or activated substances in the form of activated sludge from sewage treatment plants or waste water from a wood chip biofilter of a sewage treatment plant.

9. Method according to Claim 8, **characterised in that** the activated sludge from sewage treatment plants is selected from the group consisting of sludge of the aerobic or anaerobic treatment basin, overflow sludge of the aerobic or anaerobic treatment basin, sludge from the digestion tower, primary sludge and combinations thereof.

10. Method according to any one of the preceding claims, **characterised in that** the medium containing microorganisms was adapted to material based on cellulose and/or the metabolisation of wood extractives, precursor substances of VOCs and/or VOCs in a bioreactor prior to its use in the method according to the invention.

11. Method according to any one of the preceding claims, **characterised in that** the microorganisms contained in the medium are at least approximately 50%, preferably at least approximately 80% and even more preferably at least approximately 90% bacteria.

12. Method according to any one of the preceding claims, **characterised in that** the medium containing microorganisms contains at least one bacterial strain which is selected from the group consisting of *Enterobacter cloacae* and *Pseudomonas sp.*.

13. Method according to any one of the preceding claims, **characterised in that** the medium contains microorganisms in a total number of at least approximately $10^4$ microorganisms/ml, preferably at least approximately $10^5$ microorganisms/ml and even more preferably at least approximately $10^6$ microorganisms/ml.

14. Method according to any one of the preceding claims, **characterised in that** the precursor or intermediate product of the wooden material containing wood has a wood moisture level of at most 300%, preferably at most 250% and particularly preferably from 130 to 250% during the treatment with the medium containing microorganisms.

15. Method according to any one of the preceding claims, **characterised in that** a microorganism concentration of at least approximately $1 \times 10^3$, preferably at least approximately $1 \times 10^4$, particularly preferably at least approximately $1 \times 10^5$ or at least approximately $1 \times 10^6$ microorganisms of single-celled organisms is present per g of precursor or intermediate product containing wood during the treatment with the medium containing microorganisms.

16. Method according to any one of the preceding claims, **characterised in that** the method leads to a reduction of the content of wood extractive, precursor substances of VOCs and/or VOCs in the wooden material.

17. Method according to any one of the preceding claims, **characterised in that** the wooden material is a pressed wooden material, in particular a fibre or OSB board.

18. Method according to Claim 17, **characterised in that** the precursor or intermediate product of the wooden material containing wood are wooden particles and the method comprises at least the following steps:

**a)** bringing of wooden particles into contact with the medium containing microorganisms;
**b)** incubation of the material from step a)
**c)** drying of the pre-treated wooden particles at 80 to 400°C
**d)** gluing of the pre-treated wooden particles with a binding agent; and
**e)** pressing of the mixture into a pressed wooden material.

19. Use of a medium containing a plurality of different microorganism types in a method according to Claims 1 to 18 for the reduction of the content of wood extractive, precursor substances of VOCs and/or VOCs in wood or a wooden material.

**Revendications**

1. Procédé de préparation d'un matériau en bois, où le matériau en bois est une plaque en bois ou une pièce moulée en bois, et représente un matériau composite en particules de bois, ou un matériau en bois massif, **caractérisé en ce que** d'un produit préalable ou intermédiaire contenant du bois du matériau en bois est traité avec un milieu contenant plusieurs types différents de microorganismes, où

(i) le traitement comprend l'étape dans laquelle les différents types de microorganismes sont activement amenés ou mis en contact avec le produit préalable ou intermédiaire contenant du bois ;
(ii) le terme « traitement » ne comprend aucun processus dans lequel les microorganismes, qui étaient présents dès le départ sur ou dans le produit préalable ou intermédiaire contenant du bois du matériau en bois, n'agissent sur celui-ci ou ne commencent à décomposer celui-ci au cours du temps, et

(iii) le milieu contenant les microorganismes contient ou consiste en un biofilm et/ou des substances vivantes sous forme d'une boue activée d'installation d'épuration ou d'eaux usées d'un biofiltre en copeaux d'une station d'épuration, et contient au moins une souche bactérienne qui est choisie parmi le groupe consistant en Enterobacteriacea, Klebsiella sp., Serratia ficaria, Pantoea spp., Pseudomonadacea et Bacillus sp.

2. Procédé selon la revendication 1, **caractérisé en ce que** les microorganismes présents dans le milieu sont une microbiocénose.

3. Procédé selon la revendication 1 ou 2, **caractérisé en ce que** les microorganismes présents dans le milieu sont obtenus des eaux usées et/ou des résidus de filtre du traitement de ces eaux usées.

4. Procédé selon l'une des revendications précédentes, **caractérisé en ce que** le milieu contenant les microorganismes contient ou consiste en des eaux usées et/ou le résidu de filtre du traitement des eaux usées.

5. Procédé selon la revendication 3 ou 4, **caractérisé en ce que** les eaux usées sont choisies parmi le groupe consistant en les eaux de pluie, les eaux usées domestiques, les eaux usées communales, les eaux usées industrielles, les eaux usées des installation de traitement de l'eau et leurs combinaisons.

6. Procédé selon l'une des revendications 3 à 5, **caractérisé en ce que** les eaux usées sont des eaux usées qui ont été mises en contact avec du bois et/ou des éléments en bois, en particulier des eaux usées de l'industrie du bois et/ou du traitement du bois.

7. Procédé selon la revendication 6, **caractérisé en ce que** les eaux usées proviennent de la production de matériaux en bois, en particulier du traitement du bois par vaporisation, cuisson, défibrage ou séchage.

8. Procédé selon l'une des revendications précédentes, **caractérisé en ce que** les microorganismes présents dans le milieu sont obtenus de biofilms et/ou des substances vivantes sous forme d'une boue activée d'installation d'épuration ou d'eaux usées d'un biofiltre en copeaux d'une station d'épuration.

9. Procédé selon la revendication 8, **caractérisé en ce que** la boue activée d'installation d'épuration est choisie parmi le groupe consistant en la boue des bains de traitements aérobies et anaérobies, les boues en excès des bains de traitements aérobies et anaérobies, la boue de la tour de fermentation, la boue primaire et leurs combinaisons.

10. Procédé selon l'une des revendications précédentes, **caractérisé en ce que** le milieu contenant les microorganismes est adapté avant sa mise en oeuvre dans le procédé suivant l'invention, dans un bioréacteur aux matériaux à base de cellulose et/ou au métabolisme des extraits de bois, aux précurseurs du VOC et/ou au VOC.

11. Procédé selon l'une des revendications précédentes, **caractérisé en ce que** les microorganismes présents dans le milieu sont des bactéries pour au moins 50%, de préférence au moins 80% et de manière encore plus préférée au moins 90%.

12. Procédé selon l'une des revendications précédentes, **caractérisé en ce que** le milieu contenant les microorganismes contient au moins une souche bactérienne choisie parmi le groupe consistant en Enterobacter chloacae et Pseudomonas sp.

13. Procédé selon l'une des revendications précédentes, **caractérisé en ce que** le milieu contient des microorganismes en une quantité d'au moins environ $10^4$ microorganismes/ml, de préférence au moins environ $10^5$ microorganismes/ml et de manière encore plus préférée, au moins environ $10^6$ microorganismes/ml.

14. Procédé selon l'une des revendications précédentes, **caractérisé en ce que** le produit préalable ou intermédiaire contenant du bois du matériau en bois présente lors du traitement par le milieu contenant des microorganismes, une humidité de 300% au maximum, de préférence 250% au maximum et de manière particulièrement préférée, de 130 à 250%.

15. Procédé selon l'une des revendications précédentes, **caractérisé en ce que** lors du traitement par le milieu contenant des microorganismes présente une densité en microorganismes d'au moins environ $1 \times 10^3$, de préférence au moins

$1 \times 10^4$, de manière particulièrement préférée au moins environ $1 \times 10^5$ ou au moins environ $1 \times 10^6$ microorganismes individuels par g de produit préalable ou intermédiaire contenant du bois.

16. Procédé selon l'une des revendications précédentes, **caractérisé en ce que** le procédé conduit à une diminution de la teneur en extrait de bois, précurseurs de VOC et/ou VOC dans le matériau en bois.

17. Procédé selon l'une des revendications précédentes, **caractérisé en ce que** le matériau en bois est un matériau en bois pressé, en particulier un panneau de fibres ou OSB.

18. Procédé selon la revendication 17, **caractérisé en ce que** le produit préalable ou intermédiaire contenant du bois du matériau en bois sont des particules de bois et **en ce que** le procédé comprend au moins les étapes suivantes :

a) mise en contact des particules de bois avec le milieu contenant les microorganismes ;
b) incubation du mélange de l'étape a) ;
c) séchage des particules de bois traitées de 80 à 400°C ;
d) encollage des particules de bois traitées avec un liant, et
e) compression du mélange en un matériau en bois pressé.

19. Utilisation d'un milieu contenant plusieurs types différents de microorganismes, dans un procédé selon les revendications 1 à 18, pour diminuer la teneur en extrait de bois, précurseurs de VOC et/ou VOC dans le bois ou un matériau en bois.

Figur 1

**Hexanal**

Figur 2

**Pentanal**

Figur 3

**Hexanal**

Figur 4

**α-Pinen**

Figur 5

**β-Pinen**

Figur 6

**δ-3-Caren**

Figur 7

Hexanal

Figur 8

Σ Aldehyde

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2181818 A2
- WO 2006039914 A1
- WO 2006032267 A1
- WO 2009021702 A1
- EP 1852231 A2
- DE 102006057566 A1
- EP 2138528 A1

**In der Beschreibung aufgeführte Nicht-Patentliteratur**

- **M.J. MARTINEZ-INIGO et al.** Time course of fungal removal of lipophilic extractives from eucalyptus globulus wood. *Journal of Biotechnology,* 2000, vol. 84, 119-126
- **M.J. MARTINEZ-INIGO et al.** Biodegradability of extractives in sapwood and heartwood from scots pine by sapstain and white rot fungi. *Holzforschung,* 1999, vol. 53 (3), 247-252
- **T.A. VAN BEEK et al.** Fungal biotreatment of spruce wood with trametes versicolor for pitch control: Influence on extractive contents, pulping process parameters, paper quality and efluent toxicity. *Bioresource Technology,* 2007, vol. 98, 302-311
- BGBl. *WHG,* Juli 2009, vol. 31 (I), 2585
- *TrinkwV BGBl,* 2001, vol. I, 959 ff
- **STRATEV, D. ; GRADINGER, C. ; TERS, T. ; FACKLER, K. ; KUNCINGER, T. ; SREBOTNIK, E.** Fungal pretreatment of pine wood to reduce the emission of volatile organic compounds. *Holzforschung,* 2011, vol. 65